# EUROPEAN PATENT APPLICATION

(11) **EP 4 660 136 A2**
(43) Date of publication of application: **10.12.2025**
(21) Application number: 25211796.5
(22) Date of filing: 08.06.2022
(51) Int. Cl.: C01B 39/12

(54) **USE OF 1-METHYL-6,7-DIHYDRO-5H-CYCLOPENTA[B]PYRIDINE-1-IUM CATION AS STRUCTURE DIRECTING AGENT FOR THE PREPARATION OF EMM-64**

(30) Priority: 18.06.2021 US 202163212205 P
(62) Divisional of application: 22740681.6
(71) Applicant: ExxonMobil Technology and Engineering Company, Spring, TX 77389 (US)
(72) Inventor: BURTON, Allen, Stewartsville, NJ 08886 (US); SOLED, Stuart, Pittstown, NJ 08867 (US); VROMAN, Hilda, Piscataway, NJ 08854 (US)
(74) Representative: ExxonMobil Petroleum & Chemical BV

(57) **Abstract**

This disclosure relates to the use of 1-methyl-6,7-dihydro-5H-cyclopenta[b]pyridine-1-ium cation as structure directing agent (SDA) for the preparation of aluminosilicate RTH framework type zeolites. This disclosure further relates to a method of making an aluminosilicate molecular sieve of RTH framework type using said SDA, aluminosilicate molecular sieves of RTH framework type obtainable by said method, and uses thereof.

## Description

### CROSS REFERENCE TO RELATED APPLICATIONS

The present application claims priority to and the benefit of U.S. Provisional Application No. 63/212205 filed on June 18, 2021, which is hereby incorporated by reference in its entirety.

### TECHNICAL FIELD

This disclosure relates to the use of 1-methyl-6,7-dihydro-5H-cyclopenta[b]pyridine-1-ium cation as structure directing agent (SDA) for the preparation of zeolites. This disclosure also relates to compositions of matter, designated as EMM-64 and EMM-65, obtainable by using this SDA, as well as a method of making the same and uses thereof. This disclosure further relates to a method of making an aluminosilicate molecular sieve of RTH framework type using said SDA, RTH materials obtainable by said method, and uses thereof.

### BACKGROUND OF THE INVENTION

Molecular sieve materials, both natural and synthetic, may be used as adsorbents and have catalytic properties for hydrocarbon conversion reactions. Certain molecular sieves, such as zeolites, AlPOs, and mesoporous materials, are ordered, porous crystalline materials having a definite crystalline structure as determined by X-ray diffraction (XRD). Certain molecular sieves are ordered and produce specific identifiable XRD patterns. Within certain molecular sieve materials there may be a large number of cavities, which may be interconnected by a number of channels or pores. These cavities and pores are uniform in size within a specific molecular sieve material. Because the dimensions of these pores are such as to accept for adsorption molecules of certain dimensions while rejecting those of larger dimensions, these materials have come to be known as "molecular sieves" and are utilized in a variety of industrial processes, e.g. cracking, hydrocracking, disproportionation, alkylation, oligomerization, and isomerization.

Molecular sieves that find application in catalysis and adsorption include any of the naturally occurring or synthetic crystalline molecular sieves. Examples of these molecular sieves include large pore zeolites, intermediate pore size zeolites, and small pore zeolites. These zeolites and their isotypes are classified by the Structure Commission of the International Zeolite Association according to the rules of the IUPAC Commission on Zeolite Nomenclature. According to this classification, framework type zeolites and other crystalline microporous molecular sieves, for which a structure has been established, are assigned a three letter code and are described in the "Atlas of Zeolite Framework Types", eds. Ch. Baerlocher, L.B. McCusker, and D.H. Olson, Elsevier, Sixth Edition, 2007, which is hereby incorporated by reference. These zeolites and their isotypes are also described in http://america.iza-structure.org/IZA-SC/ftc_table.php. A large pore zeolite generally has a pore size of at least about 7 Å and includes LTL, VFI, MAZ, FAU, OFF, *BEA, and MOR framework type zeolites. Examples of large pore zeolites include mazzite, offretite, zeolite L, VPI-5, zeolite Y, zeolite X, omega, and Beta. An intermediate pore size zeolite generally has a pore size from about 5 Å to less than about 7 Å and includes, for example, MFI, MEL, EUO, MTT, MFS, AEL, AFO, HEU, FER, MWW, and TON framework type zeolites. Examples of intermediate pore size zeolites include ZSM-5, ZSM-11, ZSM-22, MCM-22, silicalite 1, and silicalite 2. A small pore size zeolite has a pore size from about 3 Å to less than about 5.0 Å and includes, for example, CHA, RTH, ERI, KFI, LEV, SOD, and LTA framework type zeolites. Examples of small pore zeolites include ZK-4, ZSM-2, SAP0-34, SAP0-35, ZK-14, SAP0-42, ZK-21, ZK-22, ZK-5, ZK-20, zeolite A, chabazite, zeolite T, and ALPO-17.

The idealized inorganic framework structure of zeolites is a framework of silicate in which all tetrahedral atoms are connected by oxygen atoms with the four next-nearest tetrahedral atoms. The term "silicate", as used herein, refers to a substance containing at least silicon and oxygen atoms that are alternately bonded to each other (i.e., -O-Si-O-Si-), and optionally including other atoms within the inorganic framework structure, including atoms such as boron, aluminum, or other metals (e.g., transition metals, such as titanium, vanadium, or zinc). Atoms other than silicon and oxygen in the framework silicate occupy a portion of the lattice sites otherwise occupied by silicon atoms in an 'all-silica' framework silicate. Thus, the term "framework silicate" as used herein refers to an atomic lattice comprising any of a silicate, borosilicate, gallosilicate, ferrisilicate, aluminosilicate, titanosilicate, zincosilicate, vanadosilicate, or the like.

The structure of the framework silicate within a given zeolite determines the size of the pores or channels that are present therein. The pore or channel size may determine the types of processes for which a given zeolite is applicable. Currently, greater than 200 unique zeolite framework silicate structures are known and recognized by the Structure Commission of the International Zeolite Association, thereby defining a range of pore geometries and orientations.

The framework silicates of zeolites are commonly characterized in terms of their ring size, wherein the ring size refers to the number of silicon atoms (or alternative atoms, such as those listed above) that are tetrahedrally coordinated with oxygen atoms in a loop to define a pore or channel within the interior of the zeolite. For example, an "8-ring" zeolite refers to a zeolite having pores or channels defined by 8 alternating tetrahedral atoms and 8 oxygen atoms in a loop. The pores or channels defined within a given zeolite may be symmetrical or asymmetrical depending upon various structural constrains that are present in the particular framework silicate.

Synthesis of molecular sieve materials typically involves hydrothermal crystallization from a synthesis mixture comprising sources of all the elements present in the zeolite such as sources of silica but also of alumina etc. In many cases a structure directing agent (SDA) is also present. Structure directing agents are compounds which are believed to promote the formation of molecular sieves and which are thought to act as templates around which certain molecular sieve structures can form and which thereby promote the formation of the desired molecular sieve. Various compounds have been used as structure directing agents including various types of quaternary ammonium cations. Typically, zeolite crystals form around structure directing agents with the structure directing agent occupying pores in the zeolite once crystallization is complete. The "as-synthesized" zeolite will therefore contain the structure directing agent in its pores so that, following crystallization, the "as-synthesized" zeolite is usually subjected to a treatment step such as a calcination step to remove the structure directing agent.

For instance, zeolite of RTH framework type was first synthesized as borosilicate, denoted as RUB-13, in the presence of 1,2,2,6,6-pentamethylpiperidine as SDA (S. Vortmann et al., Microporous Materials, 4, 111-121 (1995)). WO2001/044109 illustrates the preparation of aluminosilicate RTH, denoted as SSZ-50, using *N*-ethyl-N-methyl-5,7,7-trimethyl-2-azonium bicyclo[4.1.1]nonane cations as SDA. Aluminosilicate RTH was also prepared by using 2,6-methyl-N-methylpyridinium cations as SDA (H. Xu et al., J. Mater. Chem. A, 6, 8705-8711 (2018)). ITE-RTH structural intermediate, denoted SSZ-36, has also been reported (P. Wagner et al., J. Am. Chem. Soc., 122, 263-273 (2000)). Molecular sieves of RTH framework type have large cages with 8 x 8 channel system.

It is important to identify new structure directing agents and more efficient methods for the synthesis of molecular sieves to facilitate the preparation of new molecular sieves and/or to reduce the cost of making known zeolites. Although many different crystalline molecular sieves have been discovered, there is also a continuing need for new molecular sieves with desirable properties for gas separation and drying, organic conversion reactions, and other applications. New molecular sieves can contain novel internal pore architectures, providing enhanced selectivities in these processes.

### SUMMARY

In one aspect, the present disclosure relates to the use of 1-methyl-6,7-dihydro-5H-cyclopenta[b]pyridine-1-ium cation of Formula I: 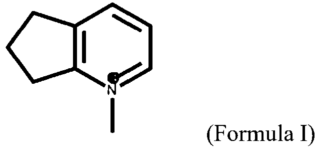 as a structure directing agent (SDA) for the preparation of zeolites.

In a second aspect, the present disclosure relates to EMM-64, a crystalline material in as-calcined form (e.g. where at least part of the SDA has been removed) and as-synthesized form (e.g. where the SDA has not been removed), as well as a method for preparing the same using 1-methyl-6,7-dihydro-5H-cyclopenta[b]pyridine-1-ium cation of Formula I as SDA, and uses thereof.

In a third aspect, the present disclosure relates to EMM-65, a crystalline material in as-calcined form (e.g. where at least part of the SDA has been removed) and as-synthesized form (e.g. where the SDA has not been removed), as well as a method for preparing the same using 1-methyl-6,7-dihydro-5H-cyclopenta[b]pyridine-1-ium cation of Formula I as SDA, and uses thereof.

In a fourth aspect, the present disclosure relates to a method of making an aluminosilicate molecular sieve of RTH framework type, using 1-methyl-6,7-dihydro-5H-cyclopenta[b]pyridine-1-ium cation of Formula I as SDA, as well as aluminosilicate molecular sieve of RTH framework type obtainable by said method and uses thereof.

In a fifth aspect, the present disclosure relates to an aluminosilicate molecular sieve of RTH framework type having 1-methyl-6,7-dihydro-5H-cyclopenta[b]pyridine-1-ium cation of Formula I within its pore structure.

In a sixth aspect, the present disclosure relates to an aluminosilicate molecular sieve of RTH framework type having a Si/Al molar ratio of less than 10.

These and other features and attributes of the present disclosure and their advantageous applications and/or uses will be apparent from the detailed description which follows. It will of course be appreciated that features described in relation to one aspect of the present invention may be incorporated into other aspects of the present invention. In particular, any two or more of the features described in this specification, including in this summary section, can be combined to form combinations of features not specifically described herein.

### DESCRIPTION OF THE DRAWINGS

Figure 1 shows the powder XRD pattern of the as-synthesized and as-calcined products of Example 2.01.
Figure 2 shows the SEM images of the as-synthesized product of Example 2.01.
Figure 3 shows the SEM images of the as-synthesized product of Example 2.06.
Figure 4 shows the SEM images of the as-synthesized products of Example 2.12.
Figure 5 shows the SEM images of the as-synthesized products of Examples 2.16, 2.17 and 2.18.
Figure 6 shows the powder XRD patterns of the as-synthesized products of Examples 2.16, 2.17 and 2.18.
Figure 7 shows the powder XRD pattern of the as-synthesized and as-calcined products of Example 3.01.
Figure 8 shows the SEM images of the as-synthesized product of Example 3.01.
Figure 9 shows the powder XRD pattern of the as-synthesized and as-calcined products of Example 3.02.
Figure 10 shows the SEM images of the as-synthesized product of Example 3.02.
Figure 11 shows the powder XRD pattern of the as-synthesized product of Example 3.04.
Figure 12 shows the SEM images of the as-synthesized product of Example 3.04.
Figure 13 shows a projection of EMM-65 along its 12-ring pores.

### DETAILED DESCRIPTION

### 1-methyl-6,7-dihydro-5H-cyclopenta[b]pyridine-1-ium cation

According to a first aspect of the present invention, it has now been found that 1-methyl-6,7-dihydro-5H-cyclopenta[b]pyridine-1-ium cation of Formula I: 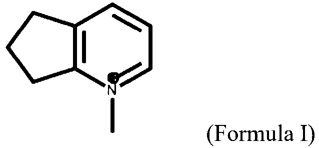 can be used as a structure directing agent (SDA) for the preparation of zeolites.

As mentioned above, it is important to identify new structure directing agents and more efficient methods for the synthesis of molecular sieves to facilitate the preparation of new molecular sieves and/or to reduce the cost of making known zeolites. It has now been found by the inventors that said cation of Formula I can suitably be used for the preparation of new crystalline materials identified as EMM-64 and EMM-65. Said crystalline materials as well as their synthesis methods and uses are disclosed herein. The present inventors have also found that said cation of Formula I can be used to prepare aluminosilicate molecular sieves of RTH framework type. Said method as well as aluminosilicate molecular sieves of RTH framework type obtainable from said method and their uses are also disclosed herein.

Said 1-methyl-6,7-dihydro-5H-cyclopenta[b]pyridine-1-ium cation may be used in a zeolite synthesis mixture in any suitable form, such as in its hydroxide form and/or halide form, e.g. chloride, bromide, iodide or fluoride form. In case the SDA is used in its hydroxide form, it also acts as a hydroxide source, providing hydroxide ions (OH) to the synthesis mixture. In case the SDA is used in a halide form, it also acts as a halide source, providing halide ions (W) to the synthesis mixture. For instance, in case the SDA is used in its fluoride form, it also acts as a fluoride source, providing fluoride ions (F) to the synthesis mixture.

1-methyl-6,7-dihydro-5H-cyclopenta[b]pyridine-1-ium cation of Formula I can be obtained by any suitable method in the art. For instance, the iodide form of the SDA may be obtained by reacting iodomethane with 6,7-dihydro-5H-cyclopenta[b]pyridine, or the bromide form of the SDA may be obtained by reacting bromomethane with 6,7-dihydro-5H-cyclopenta[b]pyridine. The hydroxide form of the cation may then be obtained by exchanging the iodide salt with hydroxide, for instance using an ion-exchange resin.

### EMM-64 crystalline material

Described herein as a second aspect of the invention are crystalline materials (e.g. zeolites) designated as EMM-64, processes of preparing these materials, and uses thereof.

The "as-synthesized" (or "as-made) EMM-64 material (i.e., before thermal treatment or other treatment to remove the SDA from the pores) typically includes the SDA, one of the components of the synthesis mixture, within its pores. The EMM-64 material, where part or all of the structure directing agent (SDA) has been removed (e.g., via thermal treatment or other treatment to remove the SDA from the pores), is an at least partially calcined or "as-calcined" EMM-64 material.

In an embodiment, the EMM-64 crystalline material of the present invention, in its as-calcined form, has an X-ray diffraction pattern including at least 9, or 10, or 11 or preferably all of the peaks in degree 2-theta selected from Table 1:

**Table 1**

| **degree 2-theta (±0.20)** | **relative intensity [100 x I/(Io)]** |
|---|---|
| 8.11 | 60-100 |
| 9.10 | 10-20 |
| 12.86 | 10-20 |
| 13.41 | 10-30 |
| 15.20 | 30-60 |
| 19.74 | 20-40 |
| 19.94 | 10-30 |
| 20.65 | 40-70 |
| 23.78 | 40-70 |
| 27.11 | 10-20 |
| 27.70 | 10-30 |
| 28.70 | 10-30 |

In another embodiment, the EMM-64 crystalline material, in its as-calcined form, may have an X-ray diffraction pattern including at least 9, or 10, or 11, or preferably all of the peaks with the degree 2-theta and d-spacing values selected from Table 1A, wherein the d-spacing values have a deviation determined based on the corresponding deviation ±0.20 degree 2-theta when converted to the corresponding values for d-spacing using Bragg's law:

**Table 1A**

| **degree 2-theta (±0.20)** | **d-spacing (Å)** | **relative intensity [100 x I/(Io)]** |
|---|---|---|
| 8.11 | 10.90 | 60-100 |
| 9.10 | 9.71 | 10-20 |
| 12.86 | 6.88 | 10-20 |
| 13.41 | 6.60 | 10-30 |
| 15.20 | 5.82 | 30-60 |
| 19.74 | 4.49 | 20-40 |
| 19.94 | 4.45 | 10-30 |
| 20.65 | 4.30 | 40-70 |
| 23.78 | 3.74 | 40-70 |
| 27.11 | 3.29 | 10-20 |
| 27.70 | 3.22 | 10-30 |
| 28.70 | 3.11 | 10-30 |

The XRD patterns with the XRD peaks described herein use Cu(K_{α}) radiation.

In yet another embodiment, the EMM-64 crystalline material (whether with SDA or where part or all of the SDA is removed) has a framework defined by the connectivities in Table 2 for the tetrahedral (T) atoms in the unit cell, where the tetrahedral (T) atoms are connected by bridging atoms.

**Table 2**

| **Topol. equiv. T-atom^{a}** | **Coordination Sequence N₁ to N₁₂** | | | | | | | | | | | |
|---|---|---|---|---|---|---|---|---|---|---|---|---|
| T1 | 4 | 11 | 24 | 39 | 66 | 98 | 129 | 162 | 209 | 263 | 327 | 385 |
| T2 | 4 | 12 | 24 | 42 | 67 | 83 | 128 | 171 | 217 | 262 | 322 | 385 |
| T3 | 4 | 12 | 25 | 41 | 64 | 95 | 132 | 168 | 209 | 265 | 330 | 384 |
| T4 | 4 | 11 | 23 | 42 | 67 | 94 | 126 | 167 | 213 | 265 | 317 | 380 |

| | | | | | | | | | | | | |
|---|---|---|---|---|---|---|---|---|---|---|---|---|
| ^{a} Topologically equivalent atom positions have the same "T-type" symbol. ^{b} Size and number of smallest ring on each angle of the T-atom (M. O'Keeffe and S.T. Hyde, *Zeolites,* 19, 370 (1997)). | | | | | | | | | | | | |

The connectivities can for instance be determined by using the public-domain software TOTOPOL by M.M.J. Treacy et al., available at http://america.iza-structure.org/IZA-SC/check_topology.php (see e.g. M.M.J. Treacy, I. Rivin, E. Balkovsky, K.H. Randall, and M.D. Foster, Microporous and Mesoporous Materials, 74, 121-132 (2004)). The tetrahedral atoms may include one or more elements selected from B, Al, Fe, Ga, Si, Ge, Sn, Ti, and Zr, or a mixture thereof. For example, the tetrahedral atoms may be selected from B, Al, or Si, or a mixture thereof. For example, the tetrahedral atoms may comprise or be Si or Al. The bridging atoms may be selected from O, N, and C, or a mixture thereof. The bridging atoms may comprise or be oxygen atoms (e.g., at least 50%, at least 60%, at least 70%, at least 80%, at least 90%, or at least 95% of the bridging atoms may be oxygen). The bridging atom C may be incorporated from the various components used to make the zeolite, e.g., the silica source. The bridging atom N may be incorporated into the zeolite after the SDA has been removed.

In a further embodiment, the EMM-64 material (whether with SDA or where part or all of the SDA is removed) may be identified as having a structure that (a) can be indexed in tetragonal space group *I 41 2 2* with unit cell dimensions a = 13.8 ± 0.20 Å and c = 18.0 ± 0.20 Å, and (b) has a 3D 8-ring channel system which is unusual in that the framework is chiral.

In one or more further embodiments, the EMM-64 crystalline material, in its as-calcined form, may have a micropore volume of 0.10 to 0.20 (e.g., 0.13) cc/g.

In one or more further embodiments, the EMM-64 crystalline material, in its as-calcined form, may be optionally represented by the molecular formula of Formula II:

(m)X₂O₃:YO₂ (Formula II),

wherein 0≤m≤0.025, X is a trivalent element, and Y is a tetravalent element. Y may be selected from Si, Ge, Sn, Ti and Zr, or a mixture thereof. For example, Y may comprise or be Si. X may be selected from Al, B, Fe and Ga, or a mixture thereof. For example, X may comprise or be Al and/or B, in particular Al. The oxygen atoms in Formula II may be replaced by carbon atoms (e.g., in the form of CH₂), which can come from sources of the components used to prepare the as-made EMM-64. The oxygen atoms in Formula II can also be replaced by nitrogen atoms, e.g., after the SDA has been removed. Formula II can represent the framework of a typical EMM-64 material, in its as-calcined form, and is not meant to be the sole representation of an EMM-64 material. The EMM-64 material may contain SDA and/or impurities after appropriate treatments to remove the SDA and impurities, which are not accounted for in Formula II. Further, Formula II does not include the protons and charge compensating ions that may be present in the as-calcined EMM-64 material.

The variable m represents the molar ratio relationship of X₂O₃ to YO₂ in Formula II. For example, when m is 0.025, the molar ratio of YO₂ to X₂O₃ is 40 and the molar ratio of Y to X is 20 (e.g., the molar ratio of Si/Al is 20). m may vary from 0 to 0.025, such as at least 0, or at least 0.0005, or at least 0.001 to at most 0.025, or at most 0.017, e.g. 0.005 to 0.025. The molar ratio of Y to X may be 20 to infinity, such as at least 20, or at least 30, and up to infinity, or up to 1000, or up to 500, e.g. 20 to 100.

The as-synthesized (i.e., before treatment to remove the SDA from the pores) EMM-64 crystalline material of the present invention may have an X-ray diffraction pattern including at least 7, or 8, or preferably all of the peaks in degree 2-theta selected from Table 3:

**Table 3**

| **degree 2-theta (±0.20)** | **relative intensity [100 x I/(Io)]** |
|---|---|
| 8.08 | 20-40 |
| 15.20 | 10-30 |
| 19.54 | 30-60 |
| 19.94 | 10-30 |
| 20.56 | 50-80 |
| 23.79 | 60-100 |
| 27.12 | 10-30 |
| 27.69 | 20-40 |
| 28.50 | 20-40 |

In another embodiment, the EMM-64 crystalline material, in its as-synthesized form, may have an X-ray diffraction pattern including at least 7, or 8, or preferably all of the peaks with the degree 2-theta and d-spacing values selected from Table 3A, wherein the d-spacing values have a deviation determined based on the corresponding deviation ±0.20 degree 2-theta when converted to the corresponding values for d-spacing using Bragg's law:

**Table 3A**

| **degree 2-theta (±0.20)** | **d-spacing (Å)** | **relative intensity [100 x I/(Io)]** |
|---|---|---|
| 8.08 | 10.93 | 20-40 |
| 15.20 | 5.83 | 10-30 |
| 19.54 | 4.54 | 30-60 |
| 19.94 | 4.45 | 10-30 |
| 20.56 | 4.32 | 50-80 |
| 23.79 | 3.74 | 60-100 |
| 27.12 | 3.29 | 10-30 |
| 27.69 | 3.22 | 20-40 |
| 28.50 | 3.13 | 20-40 |

The XRD patterns with the XRD peaks described herein use Cu(K_{α}) radiation.

In one or more embodiments, the as-synthesized EMM-64 crystalline material may be optionally represented by the molecular formula of Formula III:

(n)Q:(m)X₂O₃:YO₂ (Formula III),

wherein 0≤n≤0.2, 0≤m≤0.025, Q is 1-methyl-6,7-dihydro-5H-cyclopenta[b]pyridine-1-ium cation of Formula I, X is a trivalent element as defined for Formula II, and Y is a tetravalent element as defined for Formula II. Formula III can represent the framework of a typical as-synthesized EMM-64 material with structure directing agent (Q) and is not meant to be the sole representation of such material. The as-synthesized EMM-64 material may contain impurities which are not accounted for in Formula III. Further, Formula III does not include the protons and charge compensating ions that may be present in the as-synthesized EMM-64 material.

The variable m represents the molar ratio relationship of X₂O₃ to YO₂ in Formula III. The values for variable m in Formula III are the same as those described herein for Formula II.

The variable n represents the molar relationship of Q to YO₂ in Formula III. For example, when n is 0.1, the molar ratio of Q to YO₂ is 0.1. The molar ratio of Q to YO₂ may be from 0 to 0.2, such as from 0.02 to 0.1, e.g. 0.03 to 0.06.

The method for preparing the EMM-64 crystalline material of the second aspect of the present invention may comprise the following steps:
(a) preparing a synthesis mixture comprising water, a source of an oxide of tetravalent element (Y), optionally a source of an oxide of trivalent element (X), a structure directing agent (Q), a source of fluoride ions (F), optionally a source of hydroxide ions (OH), and optionally a source of alkali and/or alkaline earth metal element (M), wherein the structure directing agent (Q) comprises 1-methyl-6,7-dihydro-5H-cyclopenta[b]pyridine-1-ium cation of Formula I as defined above, and wherein the synthesis mixture comprises fluoride ions (F) in a F/Y molar ratio of at least 0.1;
(b) heating said synthesis mixture under crystallization conditions including a temperature of from 100°C to 200°C for a time sufficient to form crystals of said crystalline material;
(c) recovering at least a portion of the crystalline material from step (b); and
(d) optionally treating the crystalline material recovered in step (c) to remove at least part of the structure directing agent (Q).

The structure directing agent (Q) may be present in any suitable form, for example as a halide, such as a chloride or a bromide, as a hydroxide or as a nitrate, for instance in its hydroxide form. The structure directing agent (Q) may be present in the synthesis mixture in a Q/Y molar ratio of 0.01 to 1.0, such as 0.05 to 1.0, or 0.1 to 0.8, or 0.2 to 0.7, or 0.3 to 0.6, for instance more than 0.3, e.g. 0.5.

The synthesis mixture comprises at least one source of an oxide of tetravalent element Y such as Si, Ge, Sn, Ti, and/or Zr, preferably Y comprising Si, and more preferably Y being Si. Suitable sources of tetravalent element Y that can be used to prepare the synthesis mixture depend on the element Y that is selected. In embodiments where Y is silicon, Si sources (e.g. silicon oxide sources) suitable for use in the method include silicates, e.g., tetraalkyl orthosilicates such as tetramethylorthosilicate (TMOS) and tetraethylorthosilicates (TEOS), fumed silica such as Aerosil^{®} (available from Evonik), Cabosperse^{®} (available from Cabot) and Cabosil^{®} (available from DMS), precipitated silica such as Ultrasil^{®} and Sipernat^{®} 340 (available from Evonik), alkali metal silicates such as potassium silicate and sodium silicate, and aqueous colloidal suspensions of silica, for example, that sold by E.I. du Pont de Nemours under the tradename Ludox^{®} or that sold by Evonik under the tradename Aerodisp^{®}; preferably silicates, fumed silica, precipitated silica, alkali metal silicates, and colloidal silica. In embodiments where Y is germanium, suitable Ge sources include germanium oxide. In embodiments where Y is titanium, suitable Ti sources include titanium dioxide and titanium tetraalkoxides, such as titanium (IV) tetraethoxide and titanium (IV) tetrachloride. In embodiments where Y is tin, suitable Sn sources include tin chloride and tin alkoxides, such as tin ethoxide and tin isopropoxide. In embodiments where Y is zirconium, suitable Zr sources include zirconium chloride and zirconium alkoxides, such as zirconium ethoxide and zirconium isopropoxide.

The synthesis mixture optionally comprises at least one source of an oxide of trivalent element X such as Al, B, Fe and/or Ga, preferably X comprising Al and/or B, e.g. Al, and more preferably X being Al and/or B, e.g. Al. Suitable sources of trivalent element X that can be used to prepare the synthesis mixture depend on the element X that is selected. In embodiments where X is aluminum, Al sources (e.g. aluminum oxide sources) suitable for use in the method include aluminum salts, especially water-soluble salts, such as aluminum sulfate, aluminum nitrate, aluminum hydroxide, alkali metal aluminates such as sodium aluminate, and aluminum alkoxides such as aluminum isopropoxide, as well as hydrated aluminum oxides, such as boehmite, gibbsite, and pseudoboehmite, and mixtures thereof. Other aluminum sources include, but are not limited to, other water-soluble aluminum salts, sodium aluminate, aluminum alkoxides, such as aluminum isopropoxide, or aluminum metal, such as aluminum in the form of chips. In embodiments where X is boron, suitable B sources include boric acid and borate salts such as sodium tetraborate or borax and potassium tetraborate. Sources of boron tend to be more soluble than sources of aluminum in hydroxide-mediated synthesis systems. In embodiments where X is gallium, suitable Ga sources include sodium gallate, potassium gallate, and gallium salts such as gallium chloride, gallium sulfate, and gallium nitrate. In embodiments where X is iron, suitable Fe sources include iron chloride, iron nitrate, and iron oxides.

Alternatively or in addition to previously mentioned sources of Y and X (e.g. Si and Al), sources containing both Y and X elements can also be used, such as sources of Si and Al. Examples of suitable sources containing both Si and Al elements include amorphous silica-alumina gels or dried silica alumina powders, silica aluminas, clays, such as kaolin, metakaolin, and zeolites, in particular aluminosilicates such as synthetic faujasite and ultrastable faujasite, for instance Ultrastable Y (USY), beta or other large to medium pore zeolites.

The synthesis mixture may have a Y/X molar ratio of at least 10 to infinity, in particular at least 20 to infinity, e.g. 20, 30 or 100 up to 500, 1000, or infinity (i.e. no X or substantially no X). In an especially preferred embodiment of this aspect of the invention, Y is Si, optional X is Al, and the EMM-64 material is a silicate or an aluminosilicate.

The synthesis mixture also contains at least one source of fluoride ions (F). The source of fluoride ions (F) may be any compound capable of releasing fluoride ions in the molecular sieve synthesis mixture. Non-limiting examples of sources of fluoride ions (F) include hydrogen fluoride (HF); salts containing one or several fluoride ions, such as metal fluoride, preferably where the metal is sodium, potassium, calcium, magnesium, strontium or barium; ammonium fluoride (NH₄F); and ammonium bifluoride (NH₄HF₂). Especially convenient sources of fluoride ions re HF, NH₄F, and NH₄HF₂, in particular HF. The fluoride ions (F) may be present in a F/Y molar ratio of 0.1 to 1.0, such as 0.2 to 0.8, or 0.3 to 0.7, e.g. 0.3, 0.4 or 0.5.

The synthesis mixture may optionally further contain at least one source of halide ions (W), different from fluoride ions, which may be selected from the group consisting of chloride, bromide or iodide. The source of halide ions (W) may be any compound capable of releasing halide ions in the molecular sieve synthesis mixture. Non-limiting examples of sources of halide ions include hydrogen chloride, ammonium chloride, hydrogen bromide, ammonium bromide, hydrogen iodide, and ammonium iodide; salts containing one or several halide ions, such as metal halides, preferably where the metal is sodium, potassium, calcium, magnesium, strontium or barium; ammonium halides; or tetraalkylammonium halides such as tetramethylammonium halides or tetraethylammonium halides. The halide ions (W) may be present in a W/Y molar ratio of 0 to 0.2, such as 0 to 0.1, for instance less than 0.1 or even 0.

Optionally, the synthesis mixture may also contain at least one source of hydroxide ions (OH). For example, hydroxide ions can be present as a counter ion of the structure directing agent (Q) or by the use of aluminum hydroxide as a source of Al. Suitable sources of hydroxide ions can also be selected from the group consisting of alkali metal hydroxides, alkaline earth metal hydroxides, ammonium hydroxide, and mixtures thereof; such as from sodium hydroxide, potassium hydroxide, lithium hydroxide, rubidium hydroxide, calcium hydroxide, magnesium hydroxide, ammonium hydroxide, and mixtures thereof; more often sodium hydroxide, potassium hydroxide, lithium hydroxide, ammonium hydroxide, and mixtures thereof; most often sodium hydroxide and/or potassium hydroxide. The synthesis mixture may comprise the hydroxide ions source in a OH/Y molar ratio of from 0 to 1.5, such as 0 or 0.05 to 1.0 or 0.8, for instance 0.1 to 0.8 or 0.2 to 0.7, e.g. 0.5. Alternatively, the synthesis mixture may be free from a hydroxide source.

Optionally, the synthesis mixture may comprise one or more sources of alkali or alkaline earth metal cation (M). If present, M is preferably selected from the group consisting of sodium, potassium, lithium, rubidium, calcium, magnesium and mixtures thereof, preferably sodium and/or potassium, more preferably sodium. The sodium source, when present, may be sodium hydroxide, sodium aluminate, sodium silicate, sodium aluminate or sodium salts such as NaCl, NaBr or sodium nitrate. The potassium source, when present, may be potassium hydroxide, potassium aluminate, potassium silicate, a potassium salt such as KCl or KBr or potassium nitrate. The lithium source, when present, may be lithium hydroxide or lithium salts such as LiCl, LiBr, LiI, lithium nitrate, or lithium sulfate. The rubidium source, when present, may be rubidium hydroxide or rubidium salts such as RbCl, RbBr, RBI, or rubidium nitrate. The calcium source, when present, may be calcium hydroxide, for example. The magnesium source, when present, may be magnesium hydroxide, for example. The alkali or alkaline earth metal cation M may also be present in the one or more sources of a trivalent element X, such as sodium aluminate, sodium tetraborate, potassium tetraborate, sodium gallate, potassium gallate, and/or in the one or more sources of tetravalent element Y, such as potassium silicate and/or sodium silicate. The synthesis mixture may comprise the alkali or alkaline earth metal cation (M) source in a M/Y molar ratio of 0 to 1.5, such as 0 or 0.05 to 1.0 or 0.8, e.g. 0.1, 0.2 or 0.3. Alternatively, the synthesis mixture may be free from alkali or alkaline earth metal cation (M).

The synthesis may be performed with our without added nucleating seeds. If nucleating seeds are added to the synthesis mixture, the seeds may be of the same or of a different structure than EMM-64, such as EMM-64 from a previous synthesis, and may suitably present in an amount from about 0.01 ppm by weight to about 10,000 ppm by weight, based on the synthesis mixture, such as from about 100 ppm by weight to about 5,000 ppm by weight of the synthesis mixture.

The synthesis mixture typically comprises water in a H₂O/Y molar ratio of from 1 to 100, such as 1 to 75 or 1 to 50, for instance 1, 2, 3 or 4 up to 50. Depending on the nature of the components in the base mixture, the amount of solvent (e.g., water from the hydroxide solution, and optionally methanol and ethanol from the hydrolysis of silica sources) of the base mixture may be removed such that a desired solvent to Y molar ratio is achieved for the synthesis mixture. Suitable methods for reducing the solvent content may include evaporation under a static or flowing atmosphere such as ambient air, dry nitrogen, dry air, or by spray drying or freeze drying. Water may be added to the resulting mixture to achieve a desired H₂O/Y molar ratio when too much water is removed during the solvent removal process. In some examples, water removal is not necessary when the preparation have sufficient H₂O/Y molar ratio.

Carbon in the form of CH₂ may be present in the various sources of components used to prepare EMM-64, e.g., tetravalent element source (silica source) or trivalent element source (alumina source), and incorporated into the EMM-64 framework as bridging atoms. Nitrogen atoms may be incorporated into the framework of the EMM-64 material as bridging atoms after the SDA has been removed.

In one or more aspects, the synthesis mixture after solvent adjustment (e.g., where the desired water to silica ratio is achieved) may be mixed by a mechanical process such as stirring or high shear blending to assure suitable homogenization of the base mixture, for example, using dual asymmetric centrifugal mixing (e.g., a FlackTek speedmixer) with a mixing speed of 1000 to 3000 rpm (e.g., 2000 rpm).

The synthesis mixture is then subject to crystallization conditions suitable for the EMM-64 material to form. Crystallization of the EMM-64 material may be carried out under static or stirred conditions in a suitable reactor vessel, such as for example, Teflon^{®} lined or stainless steel autoclaves placed in a convection oven maintained at an appropriate temperature.

The crystallization in step (b) of the method is typically carried out at a temperature of 100°C to 200°C, such as 150°C to 170°C, for a time sufficient for crystallization to occur at the temperature used. For instance, at higher temperatures, the crystallization time may be reduced. For instance, the crystallization conditions in step (b) of the method may include heating for a period of from 1 to 100 days, such as from 1 to 50 days, for example from 1 to 30 days (e.g. 1 to 12 or 14 or 16 days, or 1 to 7 days). The crystallization time can be established by methods known in the art such as by sampling the synthesis mixture at various times and determining the yield and X-ray crystallinity of precipitated solid. Unless indicated otherwise herein, the temperature measured is the temperature of the surrounding environment of the material being heated, for example the temperature of the atmosphere in which the material is heated.

Typically, the EMM-64 product is formed in solution and can be recovered by standard means, such as by centrifugation or filtration. The separated EMM-64 product can also be washed, recovered by centrifugation or filtration and dried.

The molecular sieve of the present disclosure, when employed either as an adsorbent or as a catalyst in an organic compound conversion process may be dehydrated (e.g. dried) at least partially. This can be done by heating to a temperature in the range of 80°C to 500°C, such as 90°C to 370°C in an atmosphere such as air, nitrogen, etc., and at atmospheric, subatmospheric or superatmospheric pressures for between 30 minutes and 48 hours. Dehydration may also be performed at room temperature merely by placing the molecular sieve in a vacuum, but a longer time is required to obtain a sufficient amount of dehydration.

As a result of the crystallization process, the recovered product contains within its pores at least a portion of the structure directing agent used in the synthesis. The as-synthesized EMM-64 material recovered from step (c) may thus be subjected to thermal treatment or other treatment to remove part or all of the SDA incorporated into its pores during the synthesis. Thermal treatment (e.g., calcination) of the as-synthesized EMM-64 material typically exposes the materials to high temperatures sufficient to remove part or all of the SDA, in an atmosphere selected from air, nitrogen, ozone or a mixture thereof in a furnace. While subatmospheric pressure may be employed for the thermal treatment, atmospheric pressure is desired for reasons of convenience. The thermal treatment may be performed at a temperature up to 925°C e.g., 300°C to 700°C or 400°C to 600°C. The temperature measured is the temperature of the surrounding environment of the sample. The thermal treatment (e.g., calcination) may be carried out in a box furnace in dry air, which has been exposed to a drying tube containing drying agents that remove water from the air. The heating is usually calcined for at least 1 minute and generally no longer than 1 or at most a few days. The heating may first be carried out under a nitrogen atmosphere and then the atmosphere may be switched to air and/or ozone.

The EMM-64 material may also be subjected to an ion-exchange treatment, for example, with aqueous ammonium salts, such as ammonium nitrates, ammonium chlorides, and ammonium acetates, in order to remove remaining alkali metal cations and/or alkaline earth metal cations and to replace them with protons thereby producing the acid form of the molecular sieve. To the extent desired, the original cations of the as-synthesized material, such as alkali metal cations, can be replaced by ion exchange with other cations. Preferred replacing cations can include hydrogen ions, hydrogen precursor, e.g. ammonium ions and mixtures thereof. The ion exchange step may take place after the as-made molecular sieve is dried. The ion-exchange step may take place either before or after a calcination step.

The molecular sieve may also be subjected to other treatments such as steaming and/or washing with solvent. Such treatments are well-known to the skilled person and are carried out in order to modify the properties of the molecular sieve as desired.

### EMM-65 crystalline material

Described herein as a third aspect of the invention are crystalline materials (e.g. zeolites) designated as EMM-65, processes of preparing these materials, and uses thereof.

The "as-synthesized" (or "as-made) EMM-65 material (i.e., before thermal treatment or other treatment to remove the SDA from the pores) typically includes the SDA, one of the components of the synthesis mixture, within its pores. The EMM-65 material, where part or all of the structure directing agent (SDA) has been removed (e.g., via thermal treatment or other treatment to remove the SDA from the pores), is an at least partially calcined or "as-calcined" EMM-65 material.

In an embodiment, the EMM-65 crystalline material of the present invention, in its as-calcined form, has an X-ray diffraction pattern including at least 8, or 9, or 10, or preferably all of the peaks in degree 2-theta selected from Table 4:

**Table 4**

| **degree 2-theta (±0.20)** | **relative intensity [100 x I/(Io)]** |
|---|---|
| 6.58 | 60-100 |
| 7.91 | 50-80 |
| 8.56 | 30-60 |
| 10.30 | 20-40 |
| 19.76 | 20-40 |
| 20.52 | 10-30 |
| 21.83 | 10-30 |
| 24.07 | 20-40 |
| 24.76 | 10-20 |
| 25.55 | 10-20 |
| 26.54 | 10-20 |

In another embodiment, the EMM-65 crystalline material, in its as-calcined form, may have an X-ray diffraction pattern including at least 8, or 9, or 10, or preferably all of the peaks with the degree 2-theta and d-spacing values selected from Table 4A, wherein the d-spacing values have a deviation determined based on the corresponding deviation ±0.20 degree 2-theta when converted to the corresponding values for d-spacing using Bragg's law:

**Table 4A**

| **degree 2-theta (±0.20)** | **d-spacing (Å)** | **relative intensity [100 x I/(Io)]** |
|---|---|---|
| 6.58 | 13.42 | 60-100 |
| 7.91 | 11.17 | 50-80 |
| 8.56 | 10.32 | 30-60 |
| 10.30 | 8.58 | 20-40 |
| 19.76 | 4.49 | 20-40 |
| 20.52 | 4.32 | 10-30 |
| 21.83 | 4.07 | 10-30 |
| 24.07 | 3.69 | 20-40 |
| 24.76 | 3.59 | 10-20 |
| 25.55 | 3.48 | 10-20 |
| 26.54 | 3.36 | 10-20 |

The XRD patterns with the XRD peaks described herein use Cu(K_{α}) radiation.

In yet another embodiment, the EMM-65 crystalline material (whether with SDA or where part or all of the SDA is removed) has a framework defined by the connectivities in Table 5 for the tetrahedral (T) atoms in the unit cell, where the tetrahedral (T) atoms are connected by bridging atoms.

**Table 5**

| **Topol. equiv. T-atom^{a}** | **Coordination Sequence N₁ to N₁₂** | | | | | | | | | | | |
|---|---|---|---|---|---|---|---|---|---|---|---|---|
| T1 | 4 | 12 | 22 | 37 | 56 | 84 | 121 | 145 | 186 | 321 | 288 | 343 |
| T2 | 4 | 12 | 20 | 37 | 61 | 81 | 114 | 148 | 188 | 238 | 282 | 327 |
| T3 | 4 | 10 | 19 | 34 | 57 | 86 | 111 | 139 | 183 | 236 | 293 | 336 |
| T4 | 4 | 12 | 24 | 37 | 54 | 83 | 117 | 161 | 188 | 225 | 270 | 344 |
| T5 | 4 | 12 | 22 | 36 | 57 | 85 | 117 | 152 | 180 | 229 | 294 | 331 |
| T6 | 4 | 12 | 23 | 41 | 58 | 78 | 112 | 156 | 199 | 231 | 266 | 332 |
| T7 | 4 | 10 | 20 | 36 | 58 | 83 | 113 | 147 | 182 | 230 | 287 | 343 |

| | | | | | | | | | | | | |
|---|---|---|---|---|---|---|---|---|---|---|---|---|
| ^{a} Topologically equivalent atom positions have the same "T-type" symbol. ^{b} Size and number of smallest ring on each angle of the T-atom (M. O'Keeffe and S.T. Hyde, Zeolites, 19, 370 (1997)). | | | | | | | | | | | | |

The connectivities can for instance be determined by using the public-domain software TOTOPOL by M.M.J. Treacy et al., available at http://america.iza-structure.org/IZA-SC/check_topology.php (see e.g. M.M.J. Treacy, I. Rivin, E. Balkovsky, K.H. Randall, and M.D. Foster, Microporous and Mesoporous Materials, 74, 121-132 (2004)). The tetrahedral atoms may include one or more elements selected from B, Al, Fe, Ga, Si, Ge, Sn, Ti, and Zr, or a mixture thereof. For example, the tetrahedral atoms may be selected from B, Al, or Si, or a mixture thereof. For example, the tetrahedral atoms may comprise or be Si or Al. The bridging atoms may be selected from O, N, and C, or a mixture thereof. The bridging atoms may comprise or be oxygen atoms (e.g., at least 50%, at least 60%, at least 70%, at least 80%, at least 90%, or at least 95% of the bridging atoms may be oxygen). The bridging atom C may be incorporated from the various components used to make the zeolite, e.g., the silica source. The bridging atom N may be incorporated into the zeolite after the SDA has been removed.

In a further embodiment, the EMM-65 material (whether with SDA or where part or all of the SDA is removed) may be identified as having a structure that (a) can be indexed in orthorhombic space group *Pmmn2* with unit cell dimensions a = 5.15 ± 0.20 Å, b = 27.3 ± 0.20 Å, and c = 11.4 ± 0.20 Å, and (b) has a 1D 12-ring channel system with pore dimensions of 7.9 ± 0.20 Å by 6.0 ± 0.20 Å.

In one or more further embodiments, the EMM-65 crystalline material, in its as-calcined form, may have a micropore volume of 0.10 to 0.20 (e.g., 0.13) cc/g.

In one or more further embodiments, the EMM-65 crystalline material, in its as-calcined form, may be optionally represented by the molecular formula of Formula IV:

(m)B₂O₃:SiO₂ (Formula IV),

wherein 0.005≤m≤0.05. The oxygen atoms in Formula IV may be replaced by carbon atoms (e.g., in the form of CH₂), which can come from sources of the components used to prepare the as-made EMM-65. The oxygen atoms in Formula IV can also be replaced by nitrogen atoms, e.g., after the SDA has been removed. Formula IV can represent the framework of a typical EMM-65 material, in its as-calcined form, and is not meant to be the sole representation of an EMM-65 material. The EMM-65 material may contain SDA and/or impurities after appropriate treatments to remove the SDA and impurities, which are not accounted for in Formula IV. Further, Formula IV does not include the protons and charge compensating ions that may be present in the as-calcined EMM-65 material.

In this embodiment, the EMM-65 crystalline material is in the form of a borosilicate and the variable m represents the molar ratio relationship of B₂O₃ to SiO₂ in Formula IV. For example, when m is 0.025, the molar ratio of SiO₂ to B₂O₃ is 40 and the molar ratio of Si to B is 20. m may vary from 0.005 to 0.05, such as from at least 0.01 to 0.033, e.g. 0.025. The molar ratio of Si to B may be 10 to 100, such as 15 to 50, e.g. 20.

The as-synthesized (i.e., before treatment to remove the SDA from the pores) EMM-65 crystalline material of the present invention may have an X-ray diffraction pattern including at least 18, or 19, or 20, or preferably all of the peaks in degree 2-theta selected from

### Table 6:

**Table 6**

| **degree 2-theta (±0.20)** | **relative intensity [100 x I/(Io)]** |
|---|---|
| 6.64 | 30-60 |
| 7.97 | 40-70 |
| 8.63 | 30-60 |
| 10.35 | 10-30 |
| 15.44 | 10-30 |
| 16.24 | 10-20 |
| 18.76 | 20-40 |
| 19.85 | 20-40 |
| 20.61 | 30-60 |
| 21.93 | 30-60 |
| 23.72 | 20-40 |
| 24.18 | 60-100 |
| 24.84 | 10-30 |
| 25.64 | 30-60 |
| 26.56 | 20-40 |
| 27.60 | 10-20 |
| 28.23 | 20-40 |
| 30.84 | 10-20 |
| 33.47 | 10-20 |
| 36.04 | 20-40 |
| 37.25 | 10-30 |

In another embodiment, the EMM-65 crystalline material, in its as-synthesized form, may have an X-ray diffraction pattern including at least 18, or 19, or 20, or preferably all of the peaks with the degree 2-theta and d-spacing values selected from Table 6A, wherein the d-spacing values have a deviation determined based on the corresponding deviation ±0.20 degree 2-theta when converted to the corresponding values for d-spacing using Bragg's law:

**Table 6A**

| **degree 2-theta (±0.20)** | **d-spacing (Å)** | **relative intensity [100 x I/(Io)]** |
|---|---|---|
| 6.64 | 13.30 | 30-60 |
| 7.97 | 11.09 | 40-70 |
| 8.63 | 10.24 | 30-60 |
| 10.35 | 8.54 | 10-30 |
| 15.44 | 5.73 | 10-30 |
| 16.24 | 5.45 | 10-20 |
| 18.76 | 4.73 | 20-40 |
| 19.85 | 4.47 | 20-40 |
| 20.61 | 4.31 | 30-60 |
| 21.93 | 4.05 | 30-60 |
| 23.72 | 3.75 | 20-40 |
| 24.18 | 3.68 | 60-100 |
| 24.84 | 3.58 | 10-30 |
| 25.64 | 3.47 | 30-60 |
| 26.56 | 3.35 | 20-40 |
| 27.60 | 3.23 | 10-20 |
| 28.23 | 3.16 | 20-40 |
| 30.84 | 2.90 | 10-20 |
| 33.47 | 2.68 | 10-20 |
| 36.04 | 2.49 | 20-40 |
| 37.25 | 2.41 | 10-30 |

The XRD patterns with the XRD peaks described herein use Cu(K_{α}) radiation.

In one or more embodiments, the as-synthesized EMM-65 crystalline material may be optionally represented by the molecular formula of Formula V:

(n)Q:(m)B₂O₃: SiO₂ (Formula V),

wherein 0≤n≤0.2, 0.005<m≤0.05, and Q is 1-methyl-6,7-dihydro-5H-cyclopenta[b]pyridine-1-ium cation of Formula I. Formula V can represent the framework of a typical as-synthesized EMM-65 material with structure directing agent (Q) and is not meant to be the sole representation of such material. The as-synthesized EMM-65 material may contain impurities which are not accounted for in Formula V. Further, Formula V does not include the protons and charge compensating ions that may be present in the as-synthesized EMM-65 material.

The variable m represents the molar ratio relationship of B₂O₃ to SiO₂ in Formula V. The values for variable m in Formula V are the same as those described herein for Formula IV.

The variable n represents the molar relationship of Q to SiO₂ in Formula V. For example, when n is 0.1, the molar ratio of Q to SiO₂ is 0.1. The molar ratio of Q to SiO₂ may be from 0 to 0.2, such as from 0.02 to 0.1, e.g. 0.03 to 0.06.

The method for preparing the EMM-65 crystalline material of the third aspect of the present invention may comprise the following steps:
(a) preparing a synthesis mixture comprising water, a source of silica, a source of boron (B), a structure directing agent (Q), optionally a source of hydroxide ions (OH), and optionally a source of alkali and/or alkaline earth metal element (M), wherein the structure directing agent (Q) comprises 1-methyl-6,7-dihydro-5H-cyclopenta[b]pyridine-1-ium cation of Formula I as defined above, and wherein the synthesis mixture comprises fluoride ions (F) in a F/Si molar ratio of less than 0.1;
(b) heating said synthesis mixture under crystallization conditions including a temperature of from 100 to 200°C for a time sufficient to form crystals of said crystalline material;
(c) recovering at least a portion of the crystalline material from step (b); and
(d) optionally treating the crystalline material recovered in step (c) to remove at least part of the structure directing agent (Q).

The structure directing agent (Q) may be present in any suitable form, for example as a halide, such as a chloride or a bromide, as a hydroxide or as a nitrate, for instance in its hydroxide form. The structure directing agent (Q) may be present in the synthesis mixture in a Q/Si molar ratio of 0.01 to 1.0, such as 0.05 to 1.0, or 0.1 to 0.8, or 0.1 to 0.5, for instance 0.1 to less than 0.5, e.g. 0.2 or 0.3.

The synthesis mixture comprises at least one source of silica (e.g. silicon oxide). Suitable sources of silica for use in the method include silicates, e.g., tetraalkyl orthosilicates such as tetramethylorthosilicate (TMOS) and tetraethylorthosilicates (TEOS), fumed silica such as Aerosil^{®} (available from Evonik), Cabosperse^{®} (available from Cabot) and Cabosil^{®} (available from DMS), precipitated silica such as Ultrasil^{®} and Sipernat^{®} 340 (available from Evonik), alkali metal silicates such as potassium silicate and sodium silicate, and aqueous colloidal suspensions of silica, for example, that sold by E.I. du Pont de Nemours under the tradename Ludox^{®} or that sold by Evonik under the tradename Aerodisp^{®}; preferably silicates, fumed silica, precipitated silica, alkali metal silicates, and colloidal silica.

The synthesis mixture comprises at least one source of boron (B). Suitable sources of boron include boric acid and borate salts such as sodium tetraborate or borax and potassium tetraborate.

The synthesis mixture may have a Si/B molar ratio of at least 10 to 100, such as at least 10 or at least 15 or at least 20 up to at most 100 or at most 75 or at most 50, e.g. 20.

The synthesis mixture used for the preparation of EMM-65 material comprises fluoride ions (F) in a F/Si molar ratio of less than 0.1, such as 0 to less than 0.1, or 0 to 0.05, or 0 to 0.01, e.g. 0. In particular, the synthesis mixture used for the preparation of EMM-65 material is substantially free of fluoride ions (F). This means that no source of fluoride ions is added to the synthesis mixture in any substantial amount, e.g. the synthesis mixture has a F/Si molar ratio of less than 0.1, in particular less than 0.05, such as less than 0.01 or even less than 0.005, e.g. 0. Said fluoride ions (F), if present, may originate from any compound capable of releasing fluoride ions in the molecular sieve synthesis mixture, such as hydrogen fluoride (HF); salts containing one or several fluoride ions, such as metal fluoride, preferably where the metal is sodium, potassium, calcium, magnesium, strontium or barium; ammonium fluoride (NH₄F); and ammonium bifluoride (NH₄HF₂). Small amounts of fluoride ions (F) may also be present as impurities, for instance in the optional source of alkali or alkaline earth metal cation (M).

The synthesis mixture may optionally contain at least one source of halide ions (W), different from fluoride ions, which may be selected from the group consisting of chloride, bromide or iodide. The source of halide ions (W) may be any compound capable of releasing halide ions in the molecular sieve synthesis mixture. Non-limiting examples of sources of halide ions include hydrogen chloride, ammonium chloride, hydrogen bromide, ammonium bromide, hydrogen iodide, and ammonium iodide; salts containing one or several halide ions, such as metal halides, preferably where the metal is sodium, potassium, calcium, magnesium, strontium or barium; ammonium halides; or tetraalkylammonium halides such as tetramethylammonium halides or tetraethylammonium halides. The halide ions (W) may be present in a W/Si molar ratio of 0 to 0.2, such as 0 to 0.1, for instance less than 0.1 or even 0. In a preferred embodiment, the synthesis mixture may be substantially free from halide ions (W).

Optionally, the synthesis mixture may also contain at least one source of hydroxide ions (OH). Suitable sources of hydroxide ions can also be selected from the group consisting of alkali metal hydroxides, alkaline earth metal hydroxides, ammonium hydroxide, and mixtures thereof; such as from sodium hydroxide, potassium hydroxide, lithium hydroxide, rubidium hydroxide, calcium hydroxide, magnesium hydroxide, ammonium hydroxide, and mixtures thereof; more often sodium hydroxide, potassium hydroxide, lithium hydroxide, ammonium hydroxide, and mixtures thereof; most often sodium hydroxide and/or potassium hydroxide. The synthesis mixture may comprise the hydroxide ions source in a OH/Si molar ratio of from 0 to 1.5, such as 0 or 0.05 to 1.0 or 0.8, for instance 0.1 to 0.8 or 0.2 to 0.7, e.g. 0.3, 0.4 or 0.5. Alternatively, the synthesis mixture may be free from a hydroxide source.

Optionally, the synthesis mixture may comprise one or more sources of alkali or alkaline earth metal cation (M). If present, M is preferably selected from the group consisting of sodium, potassium, lithium, rubidium, calcium, magnesium and mixtures thereof, preferably sodium and/or potassium, more preferably sodium. The sodium source, when present, may be sodium hydroxide, sodium aluminate, sodium silicate, sodium aluminate or sodium salts such as NaCl, NaBr or sodium nitrate. The potassium source, when present, may be potassium hydroxide, potassium aluminate, potassium silicate, a potassium salt such as KCl or KBr or potassium nitrate. The lithium source, when present, may be lithium hydroxide or lithium salts such as LiCl, LiBr, LiI, lithium nitrate, or lithium sulfate. The rubidium source, when present, may be rubidium hydroxide or rubidium salts such as RbCl, RbBr, RBI, or rubidium nitrate. The calcium source, when present, may be calcium hydroxide, for example. The magnesium source, when present, may be magnesium hydroxide, for example. The alkali or alkaline earth metal cation M may also be present in the one or more sources of a trivalent element X, such as sodium aluminate, sodium tetraborate, potassium tetraborate, sodium gallate, potassium gallate, and/or in the one or more sources of tetravalent element Y, such as potassium silicate and/or sodium silicate. The synthesis mixture may comprise the alkali or alkaline earth metal cation (M) source in a M/Si molar ratio of 0 to 1.5, such as 0 or 0.05 to 1.0 or 0.8, for instance less than 0.15, e.g. 0.1. Alternatively, the synthesis mixture may be free from an alkali or alkaline earth metal cation (M) source.

The synthesis may be performed with our without added nucleating seeds. If nucleating seeds are added to the synthesis mixture, the seeds may be of the same or of a different structure than EMM-65, such as EMM-65 from a previous synthesis, and may suitably present in an amount from about 0.01 ppm by weight to about 10,000 ppm by weight, based on the synthesis mixture, such as from about 100 ppm by weight to about 5,000 ppm by weight of the synthesis mixture.

The synthesis mixture typically comprises water in a H₂O/Si molar ratio of from 1 to 100 or 1 to 75, such as 1 to 50, for instance at least 5, 10 or 20 and up to 50, e.g. 20-40. Depending on the nature of the components in the base mixture, the amount of solvent (e.g., water from the hydroxide solution, and optionally methanol and ethanol from the hydrolysis of silica sources) of the base mixture may be removed such that a desired solvent to Y molar ratio is achieved for the synthesis mixture. Suitable methods for reducing the solvent content may include evaporation under a static or flowing atmosphere such as ambient air, dry nitrogen, dry air, or by spray drying or freeze drying. Water may be added to the resulting mixture to achieve a desired H₂O/Si molar ratio when too much water is removed during the solvent removal process. In some examples, water removal is not necessary when the preparation have sufficient H₂O/Si molar ratio.

Carbon in the form of CH₂ may be present in the various sources of components used to prepare EMM-65, e.g., tetravalent element source (silica source) or trivalent element source (alumina source), and incorporated into the EMM-65 framework as bridging atoms. Nitrogen atoms may be incorporated into the framework of the EMM-65 material as bridging atoms after the SDA has been removed.

In one or more aspects, the synthesis mixture after solvent adjustment (e.g., where the desired water to silica ratio is achieved) may be mixed by a mechanical process such as stirring or high shear blending to assure suitable homogenization of the base mixture, for example, using dual asymmetric centrifugal mixing (e.g., a FlackTek speedmixer) with a mixing speed of 1000 to 3000 rpm (e.g., 2000 rpm).

The synthesis mixture is then subject to crystallization conditions suitable for the EMM-65 material to form. Crystallization of the EMM-65 material may be carried out under static or stirred conditions in a suitable reactor vessel, such as for example, polypropylene jars or Teflon^{®} lined or stainless steel autoclaves placed in a convection oven maintained at an appropriate temperature.

The crystallization in step (b) of the method is typically carried out at a temperature of 100°C to 200°C, such as 150°C to 170°C, for a time sufficient for crystallization to occur at the temperature used. For instance, at higher temperatures, the crystallization time may be reduced. For instance, the crystallization conditions in step (b) of the method may include heating for a period of from 1 to 100 days, such as from 1 to 50 days, for example from 1 to 30 days (e.g. 1 to 12 or 14 or 16 days, or 1 to 7 days). The crystallization time can be established by methods known in the art such as by sampling the synthesis mixture at various times and determining the yield and X-ray crystallinity of precipitated solid. Unless indicated otherwise herein, the temperature measured is the temperature of the surrounding environment of the material being heated, for example the temperature of the atmosphere in which the material is heated.

Typically, the EMM-65 product is formed in solution and can be recovered by standard means, such as by centrifugation or filtration. The separated EMM-65 product can also be washed, recovered by centrifugation or filtration and dried.

The molecular sieve of the present disclosure, when employed either as an adsorbent or as a catalyst in an organic compound conversion process may be dehydrated (e.g. dried) at least partially. This can be done by heating to a temperature in the range of 80°C to 500°C, such as 90°C to 370°C in an atmosphere such as air, nitrogen, etc., and at atmospheric, subatmospheric or superatmospheric pressures for between 30 minutes and 48 hours. Dehydration may also be performed at room temperature merely by placing the molecular sieve in a vacuum, but a longer time is required to obtain a sufficient amount of dehydration.

As a result of the crystallization process, the recovered product contains within its pores at least a portion of the structure directing agent used in the synthesis. The as-synthesized EMM-65 material recovered from step (c) may thus be subjected to thermal treatment or other treatment to remove part or all of the SDA incorporated into its pores during the synthesis. Thermal treatment (e.g., calcination) of the as-synthesized EMM-65 material typically exposes the materials to high temperatures sufficient to remove part or all of the SDA, in an atmosphere selected from air, nitrogen, ozone or a mixture thereof in a furnace. While subatmospheric pressure may be employed for the thermal treatment, atmospheric pressure is desired for reasons of convenience. The thermal treatment may be performed at a temperature up to 925°C e.g., 300°C to 700°C or 400°C to 600°C. The temperature measured is the temperature of the surrounding environment of the sample. The thermal treatment (e.g., calcination) may be carried out in a box furnace in dry air, which has been exposed to a drying tube containing drying agents that remove water from the air. The heating is usually calcined for at least 1 minute and generally no longer than 1 or at most a few days. The heating may first be carried out under a nitrogen atmosphere and then the atmosphere may be switched to air and/or ozone.

The EMM-65 material may also be subjected to an ion-exchange treatment, for example, with aqueous ammonium salts, such as ammonium nitrates, ammonium chlorides, and ammonium acetates, in order to remove remaining alkali metal cations and/or alkaline earth metal cations and to replace them with protons thereby producing the acid form of the molecular sieve. To the extent desired, the original cations of the as-synthesized material, such as alkali metal cations, can be replaced by ion exchange with other cations. Preferred replacing cations can include hydrogen ions, hydrogen precursor, e.g. ammonium ions and mixtures thereof. The ion exchange step may take place after the as-made molecular sieve is dried. The ion-exchange step may take place either before or after a calcination step.

Optionally, aluminum atoms may be introduced in the EMM-65 material framework (wherein part or all of the SDA has been removed) during an exchange process following the hydrothermal synthesis reaction. Framework silicates comprising boron atoms (e.g. borosilicates) may be particularly efficacious for undergoing exchange with aluminum atoms. Such exchange process may comprise exposing the EMM-65 material to an aluminum source such as an aqueous solution comprising an aluminum salt, under conditions sufficient to exchange at least a portion and up to substantially all of the boron atoms in the framework silicate with aluminum atoms. For example, an as-calcined borosilicate EMM-65 material may be converted to an aluminosilicate by heating the as-calcined EMM-65 material comprising boron with a solution of aluminum sulfate, aluminum nitrate, aluminum chloride and/or aluminum acetate (e.g. in a sealed autoclave in a convention oven at 100°C or at boiling temperature in an open system). The aluminum treated EMM-65 material may then be recovered by filtration and washed with deionized water. The resulting aluminum-exchanged EMM-65 crystalline material may be optionally represented by the molecular formula of Formula VI:

(m)Al₂O₃:SiO₂ (Formula VI),

wherein 0.005≤m≤0.05. The variable m represents the molar ratio relationship of Al₂O₃ to SiO₂ in Formula VI. The values for variable m in Formula VI are the same as those described herein for Formula IV. The oxygen atoms in Formula VI may be replaced by carbon atoms (e.g., in the form of CH₂) and/or by nitrogen atoms, the material may contain SDA and/or impurities after appropriate treatments to remove the SDA and impurities, which are not accounted for in Formula VI, and Formula VI does not include the protons and charge compensating ions that may be present. Formula VI can represent the framework of a typical aluminum-exchanged EMM-65 material, in its as-calcined form, and is not meant to be the sole representation of an aluminum-exchanged EMM-65 material.

The molecular sieve may also be subjected to other treatments such as steaming and/or washing with solvent. Such treatments are well-known to the skilled person and are carried out in order to modify the properties of the molecular sieve as desired.

### Uses of EMM-64 and EMM-65 materials

The EMM-64 and EMM-65 materials of the second and third aspects of the present invention, where part or all of the SDA has been removed, may be used as an adsorbent or as a catalyst or support for catalyst in a wide variety of hydrocarbon conversions, e.g., conversion of organic compounds to a converted product.

EMM-64 and EMM-65 materials (where part or all of the SDA is removed) may be used as an adsorbent, such as for separating at least one component from a mixture of components in the vapor or liquid phase having differential sorption characteristics with respect to the material. Therefore, at least one component can be partially or substantially totally separated from a mixture of components having differential sorption characteristics with respect to the EMM-64 or EMM-65 material by contacting the mixture with said EMM-64 or EMM-65 material to selectively sorb the one component. For instance, in a process for selectively separating one or more desired components of a feedstock from remaining components of the feedstock, the feedstock may be contacted with a sorbent that comprises the EMM-64 or EMM-65 material of the present invention at effective sorption conditions, thereby forming a sorbed product and an effluent product. One or more of the desired components are recovered from either the sorbed product or the effluent product.

EMM-64 and EMM-65 materials (where part or all of the SDA is removed) may also be used as a catalyst to catalyze a wide variety of organic compound conversion processes. Examples of chemical conversion processes, which are effectively catalyzed by the EMM-64 and EMM-65 materials described herein, either alone or in combination with one or more other catalytically active substances including other crystalline catalysts, include those requiring a catalyst with acid activity. Examples of organic conversion processes, which may be catalyzed by the EMM-64 or EMM-65 material described herein include cracking, hydrocracking, isomerization, polymerisation, reforming, hydrogenation, dehydrogenation, dewaxing, hydrodewaxing, adsorption, alkylation, transalkylation, dealkylation, hydrodecylization, disproportionation, oligomerization, dehydrocyclization and combinations thereof. The conversion of hydrocarbon feeds can take place in any convenient mode, for example in fluidized bed, moving bed, or fixed bed reactors depending on the types of process desired.

The EMM-64 and EMM-65 materials may be formulated into product compositions by combination with other materials, such as binders and/or matrix materials that provide additional hardness to the finished product. These other materials can be inert or catalytically active materials.

For instance, it may be desirable to incorporate the EMM-64 or EMM-65 material of the present invention with another material that is resistant to the temperatures and other conditions employed during use. Such materials include synthetic or naturally occurring zeolites as well as inorganic materials such as clays, silica and/or metal oxides such as alumina and mixtures thereof. The metal oxides may be either naturally occurring or in the form of gelatinous precipitates or gels including mixtures of silica and metal oxides. Use of a resistant material in conjunction with the EMM-64 or EMM-65 material, i.e., combined therewith or present during synthesis of the as-made EMM-64 or EMM-65 crystal, which crystal is active, tends to change the conversion and/or selectivity of the catalyst in certain organic conversion processes. Inactive resistant materials suitably serve as diluents to control the amount of conversion in a given process so that products can be obtained in an economic and orderly manner without employing other means for controlling the rate of reaction. These materials may be incorporated into naturally occurring clays, e.g., bentonite and kaolin, to improve the crush strength of the product under commercial operating conditions. Said inactive resistant materials, i.e., clays, oxides, etc., function as binders for the catalyst. A catalyst having good crush strength can be beneficial because in commercial use, it is desirable to prevent the catalyst from breaking down into powder-like materials.

Naturally occurring clays which may be used include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or after being subjected to calcination, acid treatment or chemical modification. Binders useful for compositing with EMM-64 and EMM-65 also include inorganic oxides selected from silica, zirconia, titania, magnesia, beryllia, alumina, yttria, gallium oxide, zinc oxide and mixtures thereof.

In addition to the foregoing materials, the EMM-64 and EMM-65 materials may be composited with a porous matrix material such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia.

These binder materials are resistant to the temperatures and other conditions, e.g., mechanical attrition, which occur in various hydrocarbon separation processes. Thus the EMM-64 and EMM-65 materials may be used in the form of an extrudate with a binder. They are typically bound by forming a pill, sphere, or extrudate. The extrudate is usually formed by extruding the molecular sieve, optionally in the presence of a binder, and drying and calcining the resulting extrudate. Further treatments such as steaming, and/or ion exchange may be carried out as required. The molecular sieve may optionally be bound with a binder having a surface area of at least 100 m²/g, for instance at least 200 m²/g, optionally at least 300 m²/g.

The relative proportions of molecular sieve and inorganic oxide matrix may vary widely, with the molecular sieve content ranging from about 1 to about 100 percent by weight and more usually, particularly when the composite is prepared in the form of extrudates, in the range of about 2 to about 95, optionally from about 20 to about 90 weight percent of the composite.

The EMM-64 and EMM-65 materials may also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such hydrogenating components may be incorporated in the composition by way of one or more of the following processes: cocrystallization; exchanged into the composition to the extent a Group IIIA element, e.g., aluminum, is in the structure; or intimately physically admixed therewith. Such components can also be impregnated in or onto the EMM-64 or EMM-65 materials, for example, by treating the molecular sieve with a hydrogenating metal-containing ion. For instance, in the case of platinum, suitable platinum compounds for this purpose include chloroplatinic acid, platinous chloride and various compounds containing a platinum amine complex. Combinations of metals and methods for their introduction can also be used.

It will be understood by a person skilled in the art that the EMM-64 and EMM-65 of the present invention may contain impurities, such as amorphous materials, unit cells having different topologies (e.g., quartz or molecular sieves of different framework type, that may or may not impact the performance of the resulting catalyst), and/or other impurities (e.g., heavy metals and/or organic hydrocarbons). Typical examples of molecular sieves of different framework type co-existing with the EMM-64 material are e.g. molecular sieves of AST and/or ITQ framework type. Typical crystalline materials co-existing with the EMM-65 material are e.g. quartz, cristobalite and/or layered phases such as kenyaite. The EMM-64 and EMM-65 materials of the present invention are preferably substantially free of impurities. The term "substantially free of impurities" (or in the alternative "substantially pure") used herein means the EMM-64 and EMM-65 materials contain a minor proportion (less than 50 wt%), preferably less than 20 wt %, more preferably less than 10 wt%, even more preferably less than 5 wt% and most preferably less than 1 wt% (e.g. less than 0.5 wt% or 0.1 wt%), of such impurities (or "non-EMM-64 and non-EMM-65 materials"), which weight percent (wt%) values are based on the combined weight of impurities and pure EMM-64 or pure EMM-65. The amount of impurities can be appropriately determined by powder XRD, rotating electron diffraction, and/or SEM/TEM (e.g. different crystal morphologies).

The EMM-64 and EMM-65 materials described herein are substantially crystalline. As used herein, the term "crystalline" refers to a crystalline solid form of a material, including, but not limited to, a single-component or multiple-component crystal form, e.g., including solvates, hydrates, and a co-crystal. Crystalline can mean having a regularly repeating and/or ordered arrangement of molecules, and possessing a distinguishable crystal lattice. For example, crystalline EMM-64 or EMM-65 can have different water or solvent content. The different crystalline lattices can be identified by solid state characterization methods such as by XRD (e.g., powder XRD). Other characterization methods known to a person of ordinary skill in the relevant art can further help identify the crystalline form as well as help determine stability and solvent/water content. As used herein, the term "substantially crystalline" means a majority (greater than 50 wt%) of the weight of a sample of a material described is crystalline and the remainder of the sample is a non-crystalline form. In one or more aspects, a substantially crystalline sample has at least 95% crystallinity (e.g., 5% of the non-crystalline form), at least 96% crystallinity (e.g., 4% of the non-crystalline form), at least 97% crystallinity (e.g., 3% of the non-crystalline form), at least 98% crystallinity (e.g., about 2% of the non-crystalline form), at least 99% crystallinity (e.g., 1% of the non-crystalline form), and 100% crystallinity (e.g., 0% of the non-crystalline form).

### Aluminosilicate molecular sieve of RTH framework type

Described herein as a fourth aspect of the invention are a method of making an aluminosilicate molecular sieve of RTH framework type (e.g. RTH zeolite), aluminosilicate molecular sieves of RTH framework type obtainable by said method, and uses thereof.

In an embodiment, said method of making aluminosilicate molecular sieve of RTH framework type comprises:
(a) preparing a synthesis mixture comprising water, a source of silica, a source of alumina, a structure directing agent (Q), optionally a source of hydroxide ions (OH), and a source of alkali and/or alkaline earth metal element (M), wherein the structure directing agent (Q) comprises 1-methyl-6,7-dihydro-5H-cyclopenta[b]pyridine-1-ium cation of Formula I as defined above, and wherein the synthesis mixture comprises fluoride ions (F) in a F/Si molar ratio of less than 0.1;
(b) heating said synthesis mixture under crystallization conditions including a temperature of from 100°C to 200°C for a time sufficient to form crystals of said molecular sieve;
(c) recovering at least a portion of the molecular sieve crystals from step (b); and
(d) optionally treating the molecular sieve crystals recovered in step (c) to remove at least part of the structure directing agent (Q).

Known methods for preparing aluminosilicate molecular sieves of RTH framework type use *N*-ethyl-*N*-methyl-5,7,7-trimethyl-azonium bicyclo [4.1.1] octane cations, or 2,6-methyl-*N*-methylpyridinium cations as SDA. It has surprisingly been found that the method of this fourth aspect allows the preparation of aluminosilicate RTH zeolites having an especially low Si/Al molar ratio. The method of this fourth aspect is also advantageous in that it allows for the preparation of aluminosilicate RTH zeolites using a non-expensive SDA, in particular a SDA that is easier to prepare and less expensive as compared to N-ethyl-N-methyl-5,7,7-trimethyl-azonium bicyclo [4.1.1] octane cations.

In this fourth aspect of the present invention, the structure directing agent (Q) may be present in any suitable form, for example as a halide, such as a chloride or a bromide, as a hydroxide or as a nitrate, for instance in its hydroxide form. The structure directing agent (Q) may be present in the synthesis mixture in a Q/Si molar ratio of 0.01 to 1.0, such as 0.05 to 1.0, or 0.1 to 0.8, or 0.1 to 0.5, for instance 0.1 to less than 0.5, e.g. 0.2 or 0.3.

The synthesis mixture comprises at least one source of silica (e.g. silicon oxide). Suitable sources of silica for use in the method include silicates, e.g., tetraalkyl orthosilicates such as tetramethylorthosilicate (TMOS) and tetraethylorthosilicates (TEOS), fumed silica such as Aerosil^{®} (available from Evonik), Cabosperse^{®} (available from Cabot) and Cabosil^{®} (available from DMS), precipitated silica such as Ultrasil^{®} and Sipernat^{®} 340 (available from Evonik), alkali metal silicates such as potassium silicate and sodium silicate, and aqueous colloidal suspensions of silica, for example, that sold by E.I. du Pont de Nemours under the tradename Ludox^{®} or that sold by Evonik under the tradename Aerodisp^{®}; preferably silicates, fumed silica, precipitated silica, alkali metal silicates, and colloidal silica.

The synthesis mixture comprises at least one source of alumina. Suitable sources of alumina for use in the method include aluminum salts, especially water-soluble salts, such as aluminum sulfate, aluminum nitrate, aluminum hydroxide, alkali metal aluminates such as sodium aluminate, and aluminum alkoxides such as aluminum isopropoxide, as well as hydrated aluminum oxides, such as boehmite, gibbsite, and pseudoboehmite, and mixtures thereof. Other aluminum sources include, but are not limited to, other water-soluble aluminum salts, sodium aluminate, aluminum alkoxides, such as aluminum isopropoxide, or aluminum metal, such as aluminum in the form of chips.

Alternatively or in addition to previously mentioned sources of Si and Al, sources containing both Si and Al elements can also be used, such as amorphous silica-alumina gels or dried silica alumina powders, silica aluminas, clays, such as kaolin, metakaolin, and zeolites, in particular aluminosilicates such as synthetic faujasite and ultrastable faujasite, for instance Ultrastable Y (USY), beta or other large to medium pore zeolites.

The synthesis mixture may have a Si/Al molar ratio of 5 to 50, such as 5 to 40, or 30, in particular 5 to less than 30, or 5 to 20, e.g. 5 or 10.

The synthesis mixture used for the preparation of zeolite of RTH framework type comprises fluoride ions (F) in a F/Si molar ratio of less than 0.1, such as 0 to less than 0.1, or 0 to 0.05, or 0 to 0.01, e.g. 0. In particular, the synthesis mixture used for the preparation of zeolite of RTH framework type is substantially free of fluoride ions (F). This means that no source of fluoride ions is added to the synthesis mixture in any substantial amount, e.g. the synthesis mixture has a F/Si molar ratio of less than 0.1, in particular less than 0.05, such as less than 0.01 or even less than 0.005, e.g. 0. Said fluoride ions (F), if present, may originate from any compound capable of releasing fluoride ions in the molecular sieve synthesis mixture, such as hydrogen fluoride (HF); salts containing one or several fluoride ions, such as metal fluoride, preferably where the metal is sodium, potassium, calcium, magnesium, strontium or barium; ammonium fluoride (NH₄F); and ammonium bifluoride (NH₄HF₂). Small amounts of fluoride ions (F) may also be present as impurities, for instance in the optional source of alkali or alkaline earth metal cation (M).

The synthesis mixture may optionally contain at least one source of halide ions (W), different from fluoride ions, which may be selected from the group consisting of chloride, bromide or iodide, e.g. chloride. The source of halide ions (W) may be any compound capable of releasing halide ions in the molecular sieve synthesis mixture. Non-limiting examples of sources of halide ions include hydrogen chloride, ammonium chloride, hydrogen bromide, ammonium bromide, hydrogen iodide, and ammonium iodide; salts containing one or several halide ions, such as metal halides, preferably where the metal is sodium, potassium, calcium, magnesium, strontium or barium; ammonium halides; or tetraalkylammonium halides such as tetramethylammonium halides or tetraethylammonium halides. The halide ions (W) may be present in a W/Si molar ratio of 0 to1.0, such as 0 to 0.5, e.g. 0.2 or 0.3. Alternatively, the synthesis mixture may be substantially free from halide ions (W).

Optionally, the synthesis mixture may also contain at least one source of hydroxide ions (OH). For example, hydroxide ions can be present as a counter ion of the structure directing agent (Q) or by the use of aluminum hydroxide as a source of Al. Suitable sources of hydroxide ions can also be selected from the group consisting of alkali metal hydroxides, alkaline earth metal hydroxides, ammonium hydroxide, and mixtures thereof; such as from sodium hydroxide, potassium hydroxide, lithium hydroxide, rubidium hydroxide, calcium hydroxide, magnesium hydroxide, ammonium hydroxide, and mixtures thereof; more often sodium hydroxide, potassium hydroxide, lithium hydroxide, ammonium hydroxide, and mixtures thereof; most often sodium hydroxide and/or potassium hydroxide. The synthesis mixture may comprise the hydroxide ions source in a OH/Si molar ratio of from 0 to 2.0, such as 0 to 1.5 or 0.1 to 1.5, for instance 0.2 to 1.3 or 0.3 to 1.0, e.g. 0.4, 0.5 or 0.6 up to 1.4, 1.2 or 1.0. Alternatively, the synthesis mixture may be free from a hydroxide source.

The synthesis mixture further comprises one or more sources of alkali or alkaline earth metal cation (M). If present, M is preferably selected from the group consisting of sodium, potassium, lithium, rubidium, calcium, magnesium and mixtures thereof, preferably sodium and/or potassium, more preferably sodium. The sodium source, when present, may be sodium hydroxide, sodium aluminate, sodium silicate, sodium aluminate or sodium salts such as NaCl, NaBr or sodium nitrate. The potassium source, when present, may be potassium hydroxide, potassium aluminate, potassium silicate, a potassium salt such as KCl or KBr or potassium nitrate. The lithium source, when present, may be lithium hydroxide or lithium salts such as LiCl, LiBr, LiI, lithium nitrate, or lithium sulfate. The rubidium source, when present, may be rubidium hydroxide or rubidium salts such as RbCl, RbBr, RBI, or rubidium nitrate. The calcium source, when present, may be calcium hydroxide, for example. The magnesium source, when present, may be magnesium hydroxide, for example. The alkali or alkaline earth metal cation M may also be present in the one or more sources of silica or alumina, such as sodium aluminate, potassium silicate and/or sodium silicate. The synthesis mixture may comprise the alkali or alkaline earth metal cation (M) source in a M/Si molar ratio of 0.1 to 2.0, such as 0.1 to 1.5, for instance 0.15 to 1.0, e.g. 0.15, 0.2 or 0.3 up to 1.0 or 0.7.

The synthesis may be performed with our without added nucleating seeds. If nucleating seeds are added to the synthesis mixture, the seeds may be of the same or of a different structure than molecular sieve of RTH framework type, for instance RTH zeolite from a previous (same or different) synthesis, and may suitably present in an amount from about 0.01 ppm by weight to about 10,000 ppm by weight, based on the synthesis mixture, such as from about 100 ppm by weight to about 5,000 ppm by weight of the synthesis mixture.

The synthesis mixture typically comprises water in a H₂O/Si molar ratio of from 1 to 100, such as 2 to 80, for instance at least 10, 20, 30 or 40 and up to 80 or 70, e.g. 40-70. Depending on the nature of the components in the base mixture, the amount of solvent (e.g., water from the hydroxide solution, and optionally methanol and ethanol from the hydrolysis of silica sources) of the base mixture may be removed such that a desired solvent to Si molar ratio is achieved for the synthesis mixture. Suitable methods for reducing the solvent content may include evaporation under a static or flowing atmosphere such as ambient air, dry nitrogen, dry air, or by spray drying or freeze drying. Water may be added to the resulting mixture to achieve a desired H₂O/Si molar ratio when too much water is removed during the solvent removal process. In some examples, water removal is not necessary when the preparation have sufficient H₂O/Si molar ratio.

Carbon in the form of CH₂ may be present in the various sources of components used to prepare the RTH zeolite, e.g., tetravalent element source (silica source) or trivalent element source (alumina source), and incorporated into the RTH zeolite framework as bridging atoms. Nitrogen atoms may be incorporated into the framework of the RTH zeolite as bridging atoms after the SDA has been removed.

In one or more aspects, the synthesis mixture after solvent adjustment (e.g., where the desired water to silica ratio is achieved) may be mixed by a mechanical process such as stirring or high shear blending to assure suitable homogenization of the base mixture, for example, using dual asymmetric centrifugal mixing (e.g., a FlackTek speedmixer) with a mixing speed of 1000 rpm to 3000 rpm (e.g., 2000 rpm).

The synthesis mixture is then subject to crystallization conditions suitable for the molecular sieve of RTH framework type to form. Crystallization of the RTH molecular sieve may be carried out under static or stirred conditions in a suitable reactor vessel, such as for example, polypropylene jars or Teflon^{®} lined or stainless steel autoclaves placed in a convection oven maintained at an appropriate temperature.

The crystallization in step (b) of the method is typically carried out at a temperature of 100°C to 200°C, such as 150°C to 170°C, for a time sufficient for crystallization to occur at the temperature used. For instance, at higher temperatures, the crystallization time may be reduced. For instance, the crystallization conditions in step (b) of the method may include heating for a period of from 1 to 100 days, such as from 1 to 50 days, for example from 1 to 30 days (e.g. 1 to 12 or 14 or 16 days, or 1 to 7 days). The crystallization time can be established by methods known in the art such as by sampling the synthesis mixture at various times and determining the yield and X-ray crystallinity of precipitated solid. Unless indicated otherwise herein, the temperature measured is the temperature of the surrounding environment of the material being heated, for example the temperature of the atmosphere in which the material is heated.

Typically, the molecular sieve of RTH framework type is formed in solution and can be recovered by standard means, such as by centrifugation or filtration. The separated RTH molecular sieve can also be washed, recovered by centrifugation or filtration and dried.

The molecular sieve of the present disclosure, when employed either as an adsorbent or as a catalyst in an organic compound conversion process may be dehydrated (e.g. dried) at least partially. This can be done by heating to a temperature in the range of 80°C to 500°C, such as 90°C to 370°C in an atmosphere such as air, nitrogen, etc., and at atmospheric, subatmospheric or superatmospheric pressures for between 30 minutes and 48 hours. Dehydration may also be performed at room temperature merely by placing the molecular sieve in a vacuum, but a longer time is required to obtain a sufficient amount of dehydration.

As a result of the crystallization process, the recovered product contains within its pores at least a portion of the structure directing agent used in the synthesis. The as-synthesized RTH zeolite recovered from step (c) may thus be subjected to thermal treatment or other treatment to remove part or all of the SDA incorporated into its pores during the synthesis. Thermal treatment (e.g., calcination) of the as-synthesized RTH zeolite typically exposes the materials to high temperatures sufficient to remove part or all of the SDA, in an atmosphere selected from air, nitrogen, ozone or a mixture thereof in a furnace. While subatmospheric pressure may be employed for the thermal treatment, atmospheric pressure is desired for reasons of convenience. The thermal treatment may be performed at a temperature up to 925°C e.g., 300°C to 700°C or 400°C to 600°C. The temperature measured is the temperature of the surrounding environment of the sample. The thermal treatment (e.g., calcination) may be carried out in a box furnace in dry air, which has been exposed to a drying tube containing drying agents that remove water from the air. The heating is usually calcined for at least 1 minute and generally no longer than 1 or at most a few days. The heating may first be carried out under a nitrogen atmosphere and then the atmosphere may be switched to air and/or ozone.

The molecular sieve of RTH framework type may also be subjected to an ion-exchange treatment, for example, with aqueous ammonium salts, such as ammonium nitrates, ammonium chlorides, and ammonium acetates, in order to remove remaining alkali metal cations and/or alkaline earth metal cations and to replace them with protons thereby producing the acid form of the molecular sieve. To the extent desired, the original cations of the as-synthesized material, such as alkali metal cations, can be replaced by ion exchange with other cations. Preferred replacing cations can include hydrogen ions, hydrogen precursor, e.g. ammonium ions and mixtures thereof. The ion exchange step may take place after the as-made molecular sieve is dried. The ion-exchange step may take place either before or after a calcination step.

The molecular sieve of RTH framework type may also be subjected to other treatments such as steaming and/or washing with solvent. Such treatments are well-known to the skilled person and are carried out in order to modify the properties of the molecular sieve as desired.

Aluminosilicate molecular sieves of RTH framework type are characterized by an X-ray diffraction pattern as disclosed in WO2001/044109, which is incorporated herein by reference in its entirety. More particularly, as-calcined RTH aluminosilicates have a powder X-ray diffraction pattern including the characteristic peaks with the degree 2-theta and d-spacing values shown in Table 7, wherein the d-spacing values have a deviation determined based on the corresponding deviation ±0.20 degree 2-theta when converted to the corresponding values for d-spacing using Bragg's law:

**Table 7**

| **degree 2-theta (±0.20)** | **d-spacing (Å)** | **relative intensity [100 x I/(Io)]** |
|---|---|---|
| 8.45 | 10.5 | 35-100 |
| 8.95 | 9.87 | 40-100 |
| 10.1 | 8.76 | 10-80 |
| 17.6 | 5.04 | 10-60 |
| 18.55 | 4.78 | 5-60 |
| 19.60 | 4.53 | 20-60 |
| 19.86 | 4.47 | 20-60 |
| 22.95 | 3.87 | 5-25 |
| 24.8 | 3.19 | 5-75 |
| 30.45 | 2.93 | 5-25 |
| 32.25 | 2.78 | 5-15 |
| 37.0 | 2.43 | 1-10 |

The XRD patterns with the XRD peaks described herein use Cu(K_{α}) radiation.

In a further embodiment, the present invention relates to an aluminosilicate molecular sieve of RTH framework type obtainable by (or obtained by) the method of the fourth aspect disclosed herein.

A fifth aspect of the present invention relates to an aluminosilicate molecular sieve of RTH framework type, having 1-methyl-6,7-dihydro-5H-cyclopenta[b]pyridine-1-ium cation of Formula I as defined above within its pore structure. Said RTH zeolite may be represented by the molecular formula of Formula VII:

(n)Q:(m)Al₂O₃:SiO₂ (Formula VII),

wherein 0≤n≤0.2, 0.01<m≤0.10, and Q is 1-methyl-6,7-dihydro-5H-cyclopenta[b]pyridine-1-ium cation of Formula I. Formula VII represent the framework of a typical aluminosilicate RTH zeolite having structure directing agent (Q) within its pore structure and is not meant to be the sole representation of such material. The RTH zeolite material may contain impurities which are not accounted for in Formula VII. Further, Formula VII does not include the protons and charge compensating ions that may be present in the RTH zeolite material.

The variable m represents the molar ratio relationship of Al₂O₃ to SiO₂ in Formula VII. For example, when m is 0.10, the molar ratio of SiO₂ to Al₂O₃ is 10 and the Si/Al molar ratio is 5. m may vary from 0.01 to 0.1, such as from 0.0125 to 0.1, e.g. from 0.025 to 0.1. The molar ratio of Si to Al may be 5 to 50, such as 5 to 40 or 30, in particular 5 to less than 30, for instance 5 to 20 or even 5 to 15, e.g. 5 to 10 or even 5 to less than 10.

The variable n represents the molar relationship of Q to SiO₂ in Formula VII. For example, when n is 0.1, the molar ratio of Q to Si₂ is 0.1. The molar ratio of Q to SiO₂ may be from 0 to 0.2, such as from 0.02 to 0.1, e.g. 0.03 to 0.06.

A sixth aspect of the present invention relates to an aluminosilicate molecular sieve of RTH framework type having an especially low Si/Al molar ratio, in particular a Si/Al molar ratio of less than 10, e.g. from 5 to less than 10, such as 7 or 8. Such aluminosilicate molecular sieve of RTH framework type may be represented by the molecular formula of Formula VIII:

(m)Al₂O₃:SiO₂ (Formula VIII),

wherein 0.05<m≤0.10. The variable m represents the molar ratio relationship of Al₂O₃ to SiO₂ in Formula VIII. For example, when m is 0.1, the molar ratio of SiO₂ to Al₂O₃ is 10 and the Si/Al molar ratio is 5. The oxygen atoms in Formula VIII may be replaced by carbon atoms (e.g., in the form of CH₂), which can come from sources of the components used to prepare the as-made RTH zeolite. The oxygen atoms in Formula VIII can also be replaced by nitrogen atoms, e.g., after the SDA has been removed. Formula VIII can represent the framework of a typical RTH zeolite, in its as-calcined form, and is not meant to be the sole representation of a RTH zeolite. The RTH zeolite may contain SDA and/or impurities after appropriate treatments to remove the SDA and impurities, which are not accounted for in Formula VIII. Further, Formula VIII does not include the protons and charge compensating ions that may be present in the as-calcined RTH zeolite.

### Uses of aluminosilicate molecular sieve of RTH framework type

The aluminosilicate molecular sieve of RTH framework type obtainable by the method of the fourth aspect of the present invention, where part or all of the SDA has been removed, or as defined in the fifth and/or sixth aspects of the present invention, may be used as an adsorbent or as a catalyst or support for catalyst in a wide variety of hydrocarbon conversions, e.g., conversion of organic compounds to a converted product.

Aluminosilicate RTH zeolite materials (where part or all of the SDA is removed) may be used as an adsorbent, such as for separating at least one component from a mixture of components in the vapor or liquid phase having differential sorption characteristics with respect to the material. Therefore, at least one component can be partially or substantially totally separated from a mixture of components having differential sorption characteristics with respect to the aluminosilicate RTH zeolite material by contacting the mixture with said RTH material to selectively sorb the one component. For instance, in a process for selectively separating one or more desired components of a feedstock from remaining components of the feedstock, the feedstock may be contacted with a sorbent that comprises the aluminosilicate RTH zeolite of the present invention at effective sorption conditions, thereby forming a sorbed product and an effluent product. One or more of the desired components are recovered from either the sorbed product or the effluent product.

Aluminosilicate RTH zeolite (where part or all of the SDA is removed) may also be used as a catalyst to catalyze a wide variety of organic compound conversion processes. Examples of chemical conversion processes, which are effectively catalyzed by the aluminosilicate RTH zeolite described herein, either alone or in combination with one or more other catalytically active substances including other crystalline catalysts, include those requiring a catalyst with acid activity. Examples of organic conversion processes, which may be catalyzed by the aluminosilicate RTH zeolite described herein include cracking, hydrocracking, reforming, hydrogenation, dehydrogenation, dewaxing, adsorption, alkylation, oligomerization, conversion methanol to olefins, deNOx applications, and combinations thereof. The conversion of hydrocarbon feeds can take place in any convenient mode, for example in fluidized bed, moving bed, or fixed bed reactors depending on the types of process desired.

The aluminosilicate RTH zeolite may be formulated into product compositions by combination with other materials, such as binders and/or matrix materials that provide additional hardness to the finished product. These other materials can be inert or catalytically active materials.

For instance, it may be desirable to incorporate the aluminosilicate RTH zeolite of the fifth or sixth aspect of the present invention or obtainable by the process of the fourth aspect of the present invention with another material that is resistant to the temperatures and other conditions employed during use. Such materials include synthetic or naturally occurring zeolites as well as inorganic materials such as clays, silica and/or metal oxides such as alumina and mixtures thereof. The metal oxides may be either naturally occurring or in the form of gelatinous precipitates or gels including mixtures of silica and metal oxides. Use of a resistant material in conjunction with the aluminosilicate RTH zeolite, i.e., combined therewith or present during synthesis of the as-made aluminosilicate RTH zeolite, which crystal is active, tends to change the conversion and/or selectivity of the catalyst in certain organic conversion processes. Inactive resistant materials suitably serve as diluents to control the amount of conversion in a given process so that products can be obtained in an economic and orderly manner without employing other means for controlling the rate of reaction. These materials may be incorporated into naturally occurring clays, e.g., bentonite and kaolin, to improve the crush strength of the product under commercial operating conditions. Said inactive resistant materials, i.e., clays, oxides, etc., function as binders for the catalyst. A catalyst having good crush strength can be beneficial because in commercial use, it is desirable to prevent the catalyst from breaking down into powder-like materials.

Naturally occurring clays which may be used include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or after being subjected to calcination, acid treatment or chemical modification. Binders useful for compositing with aluminosilicate RTH zeolite also include inorganic oxides selected from silica, zirconia, titania, magnesia, beryllia, alumina, yttria, gallium oxide, zinc oxide and mixtures thereof.

In addition to the foregoing materials, the aluminosilicate RTH zeolite may be composited with a porous matrix material such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia.

These binder materials are resistant to the temperatures and other conditions, e.g., mechanical attrition, which occur in various hydrocarbon separation processes. Thus the aluminosilicate RTH zeolite may be used in the form of an extrudate with a binder. They are typically bound by forming a pill, sphere, or extrudate. The extrudate is usually formed by extruding the molecular sieve, optionally in the presence of a binder, and drying and calcining the resulting extrudate. Further treatments such as steaming, and/or ion exchange may be carried out as required. The molecular sieve may optionally be bound with a binder having a surface area of at least 100 m²/g, for instance at least 200 m²/g, optionally at least 300 m²/g.

The relative proportions of molecular sieve and inorganic oxide matrix may vary widely, with the molecular sieve content ranging from about 1 to about 100 percent by weight and more usually, particularly when the composite is prepared in the form of extrudates, in the range of about 2 to about 95, optionally from about 20 to about 90 weight percent of the composite.

The aluminosilicate RTH zeolite may also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such hydrogenating components may be incorporated in the composition by way of one or more of the following processes: cocrystallization; exchanged into the composition to the extent a Group IIIA element, e.g., aluminum, is in the structure; or intimately physically admixed therewith. Such components can also be impregnated in or onto the aluminosilicate RTH zeolite, for example, by treating the molecular sieve with a hydrogenating metal-containing ion. For instance, in the case of platinum, suitable platinum compounds for this purpose include chloroplatinic acid, platinous chloride and various compounds containing a platinum amine complex. Combinations of metals and methods for their introduction can also be used.

It will be understood by a person skilled in the art that the aluminosilicate RTH zeolite of the fifth or sixth aspect of the present invention or obtainable by the method of the fourth aspect of the present invention may contain impurities, such as amorphous materials, unit cells having different topologies (e.g., quartz or molecular sieves of different framework type, that may or may not impact the performance of the resulting catalyst), and/or other impurities (e.g., heavy metals and/or organic hydrocarbons). Typical examples of crystalline materials co-existing with the aluminosilicate RTH zeolite are e.g. quartz, mordenite, cristobalite, zeolite P, and/or molecular sieves of ANA framework type. The aluminosilicate RTH zeolite is preferably substantially free of impurities. The term "substantially free of impurities" (or in the alternative "substantially pure") used herein means the aluminosilicate RTH zeolite contains a minor proportion (less than 50 wt%), preferably less than 20 wt%, more preferably less than 10 wt%, even more preferably less than 5 wt% and most preferably less than 1 wt% (e.g. less than 0.5 wt% or 0.1 wt%), of such impurities (or "non-RTH materials"), which weight percent (wt%) values are based on the combined weight of impurities and pure aluminosilicate RTH zeolite. The amount of impurities can be appropriately determined by powder XRD, rotating electron diffraction, and/or SEM / TEM (e.g. different crystal morphologies).

The aluminosilicate RTH zeolite described herein are substantially crystalline. As used herein, the term "crystalline" refers to a crystalline solid form of a material, including, but not limited to, a single-component or multiple-component crystal form, e.g., including solvates, hydrates, and a co-crystal. Crystalline can mean having a regularly repeating and/or ordered arrangement of molecules, and possessing a distinguishable crystal lattice. For example, aluminosilicate RTH zeolite can have different water or solvent content. The different crystalline lattices can be identified by solid state characterization methods such as by XRD (e.g., powder XRD). Other characterization methods known to a person of ordinary skill in the relevant art can further help identify the crystalline form as well as help determine stability and solvent/water content. As used herein, the term "substantially crystalline" means a majority (greater than 50 wt%) of the weight of a sample of a material described is crystalline and the remainder of the sample is a non-crystalline form. In one or more aspects, a substantially crystalline sample has at least 95% crystallinity (e.g., 5% of the non-crystalline form), at least 96% crystallinity (e.g., 4% of the non-crystalline form), at least 97% crystallinity (e.g., 3% of the non-crystalline form), at least 98% crystallinity (e.g., about 2% of the non-crystalline form), at least 99% crystallinity (e.g., 1% of the non-crystalline form), and 100% crystallinity (e.g., 0% of the non-crystalline form).

Aspects of the disclosure are described in greater detail by way of specific examples. The following examples are offered for illustrative purposes, and are not intended to limit the disclosure in any manner. Those of skill in the relevant art will readily recognize a variety of parameters can be changed or modified to yield essentially the same results.

### EXAMPLES

The present invention is further illustrated below without limiting the scope thereto.

In these examples, the X-ray diffraction (XRD) patterns of the as-synthesized and as-calcined materials were recorded on an X-Ray Powder Diffractometer (Bruker DaVinci D8 Discovery instrument) in continuous mode using a Cu Kα radiation, Bragg-Bentano geometry with Vantec 500 detector, in the 2*θ* range of 4 to 36 degrees. The interplanar spacings, d-spacings, were calculated in Angstrom units, and the relative intensities of the lines, I/I_{O} is the ratio of the peak intensity to that of the intensity of the strongest line, above background. The intensities are uncorrected for Lorentz and polarization effects. The location of the diffraction peaks in 2-theta, and the relative peak area intensities of the lines, I/I(o), where Io is the intensity of the strongest line, above background, were determined with the MDI Jade peak search algorithm. It should be understood that diffraction data listed as single lines may consist of multiple overlapping lines which under certain conditions, such as differences in crystallographic changes, may appear as resolved or partially resolved lines. Typically, crystallographic changes can include minor changes in unit cell parameters and/or a change in crystal symmetry, without a change in the structure. These minor effects, including changes in relative intensities, can also occur as a result of differences in cation content, framework composition, nature and degree of pore filling, crystal size and shape, preferred orientation and thermal and/or hydrothermal history.

The scanning electron microscopy (SEM) images of the as-synthesized materials were obtained on a Hitachi 4800 Scanning Electron Microscope. SEM images were used to aid assessment of product purity. The presence of obviously different crystal morphologies in a SEM image can be an indication of impurities in the form of other crystalline materials. Such an approximate analysis can be especially useful in identifying the presence of formation of relatively minor amounts of crystalline impurities which may not be identifiable on product XRD patterns.

The following measurements were conducted on samples that were ion-exchanged and calcined. For each sample subjected to ion-exchange and calcination, the procedure used was as follows: the as-prepared sample was washed two times with a 1M ammonium nitrate solution and then calcined at 500°C for 16 hours.

The overall BET surface area (S_{BET}) of the materials was determined by the BET method as described by S. Brunauer, P.H. Emmett and E. Teller, J. Am. Chem. Soc., 1938, 60, 309, incorporated herein by reference, using nitrogen adsorption-desorption at liquid nitrogen temperature.

The micropore volume (V_{micro}) of the materials can be determined using methods known in the relevant art. For example, the micropore volume of the materials can be measured with nitrogen physisorption, and the data can be analyzed by the t-plot method described in Lippens, B.C. et al., "Studies on pore system in catalysts: V. The t method", J. Catal., 4, 319 (1965), which describes micropore volume method and is incorporated herein by reference.

Alpha value is a measure of the cracking activity of a catalyst and is described in U.S. Pat. No. 3,354,078 and in the Journal of Catalysis, Vol. 4, p. 527 (1965); Vol. 6, p. 278 (1966); and Vol. 61, p. 395 (1980), each incorporated herein by reference as to that description. The experimental conditions of the test used herein include a constant temperature of 538°C and a variable flow rate as described in detail in the Journal of Catalysis, Vol. 61, p. 395.

### Example 1 - Synthesis of 1-methyl-6,7-dihydro-5H-cyclopenta[b]pyridine-1-ium hydroxide

In a 500-mL round-bottom flask, 25.0 g of 6,7-dihydro-5H-cyclopenta[b]pyridine was dissolved in 200 mL acetone. At ambient conditions, 41.76 g of iodomethane was then slowly delivered dropwise to the flask via a dropping funnel. The next day ethyl ether was added to precipitate out any dissolved solids. The solids were then recovered by filtration and rinsed with ether and allowed to dry. The yield of recovered product was 52 g. The purity of the product was verified by ¹H and ¹³C NMR.

The halide salt of 1-methyl-6,7-dihydro-5H-cyclopenta[b]pyridine-1-ium was then ion-exchanged into the hydroxide form by dissolving it in water and adding it to a 2-fold excess of Dowex LC NG hydroxide exchange resin. The resin was then removed by filtration and washed with deionized water to remove the product from the resin. The aqueous fractions were then combined and concentrated under reduced pressure at about 60°C. The concentration of this aqueous solution was determined as 7.4 wt% by titration with a standard solution of 0.1 M HCl.

### Example 2 - Synthesis of EMM-64

The molar ratios and conditions used for the syntheses of Examples 2.01-2.21, as well as the resulting products, are detailed below and summarized in Table 8.

### Example 2.01 - Synthesis of EMM-64

EMM-64 was first observed from a synthesis performed in a 1.5 mL stainless steel reactor tumbled at 150°C for 10 days, from a synthesis mixture having the following composition in terms of molar ratios: H₂O/Si = 4, Q/Si = 0.50, OH/Si = 0.50, HF/Si = 0.50, using 1-methyl-6,7-dihydro-5H-cyclopenta[b]pyridine-1-ium hydroxide as prepared in Example 1 as structure directing agent (Q) (7.4 wt% solution). The silica source was tetramethylorthosilicate (TMOS, > 99 wt%). HF was in the form of a 15 wt% solution.

The product was isolated by centrifugation, resuspension in deionized water, and then another centrifugation. This process was repeated three times and then the product was dried at 90°C. The as-synthesized material was then calcined to 600°C within a box furnace via the following procedure. The sample was exposed to flowing nitrogen for two hours at room temperature, followed by a ramp from room temperature to 400°C over a two-hour period while remaining under nitrogen flow. The temperature then remained at 400°C for 15 minutes and then the atmosphere was switched from flowing nitrogen to flowing dried air. The temperature was then ramped from 400°C to 600°C over a 1-hour period. The temperature remained at 600°C for 2 hours and then the box furnace was allowed to cool.

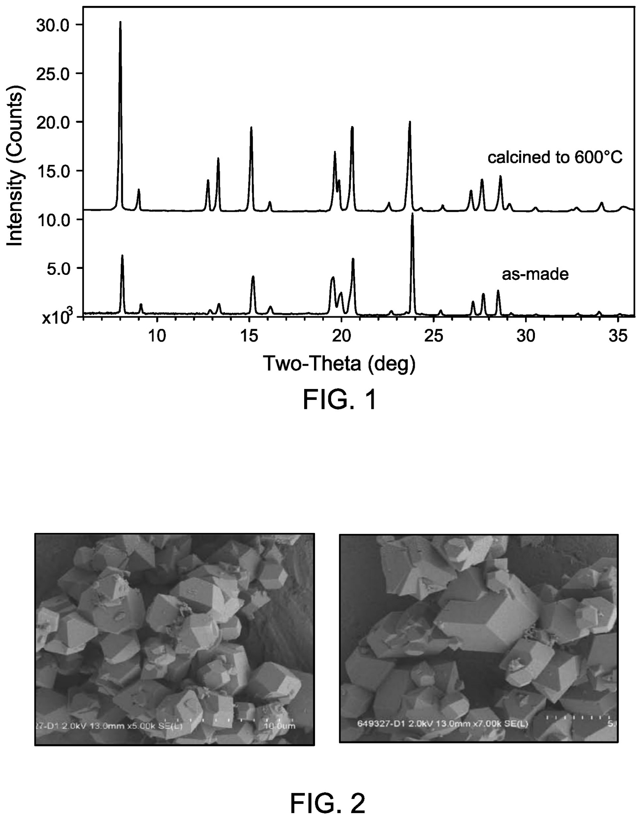

XRD analysis of the as-synthesized and as-calcined materials showed the material to have unique powder XRD patterns which could not be matched with any known zeolite and were designated as pure as-synthesized and as-calcined EMM-64 products. Figure 1 shows the powder XRD of the as-synthesized and as-calcined materials. Figure 2 shows SEM images of the as-synthesized product.

### Example 2.02 - Larger scale synthesis of EMM-64

This Example was a larger-scale reproduction of Example 2.01, synthesized in a 10 mL Teflon^{®} reactor. 17.4 g 1-methyl-6,7-dihydro-5H-cyclopenta[b]pyridin-1-ium hydroxide (7.4 wt% solution) was added to 2.6g TMOS (>99 wt%). The mixture was placed on a stir plate and allowed to stir for 1 hour before placing it in a vacuum oven at about 50°C. The reactor was left in vacuum oven and weighed periodically until 16.15 g of water were evaporated. The vessel was then removed from the vacuum oven and 0.262 g water and 1.14 g HF (15 wt% solution) were added to the reactor. The Teflon reactor was then placed in the metal housing and heated at 150°C for 14 days in a tumbling oven (about 45 rpm). After 14 days, the reactor was discharged and the product was collected using centrifugation and washing three times with distilled water (200 mL). The product was dried at 90°C in a vented drying oven. XRD patterns of the as-synthesized and as-calcined materials showed the product to be pure EMM-64.

### Examples 2.03-2.05 - Synthesis of EMM-64 with varying H₂O/Si molar ratios

These Examples were carried out under similar conditions as Example 2.02 except for varying H₂O/Si molar ratios of respectively 20, 30 and 50. Each Experiment produced pure EMM-64 after 14 days of heating at 150°C, as identified by their XRD patterns. Examples 2.06-2.11 - Synthesis of EMM-64 at 130°C, with varying H₂O/Si molar ratios

These Examples were carried out under similar conditions as Example 2.02 except at 130°C, with varying H₂O/Si molar ratios from 4 to 50, at a scale of 3.6 g TMOS reactant.

At H₂O/Si molar ratios of 7, 10 and 20, Examples 2.08, 2.06 and 2.09 produced pure EMM-64, as identified by their XRD patterns. At a low H₂O/Si molar ratio of 4, Example 2.07 produced mostly amorphous product with minor EMM-64 after 27 days at 130°C. At higher H₂O/Si molar ratios of 30 and 50, Examples 2.10 and 2.11 respectively produced EMM-64 with minor amounts of amorphous material after 27 days at 130°C.

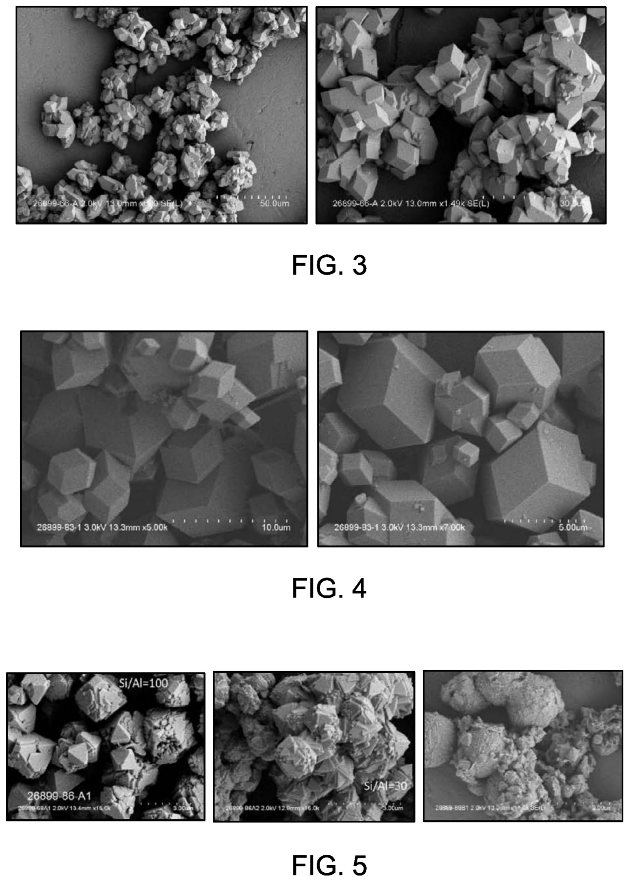

Figure 3 shows SEM images of the as-synthesized product of Example 2.06. Tables 9 and 10 below show the list of peaks and intensities for the as-calcined and as-made EMM-64 products of Example 2.06. The powder diffraction data of the as-synthesized and as-calcined EMM-64 products could be indexed in tetragonal space group *I 41 2 2* with unit cell dimensions a = 13.8 ± 0.20 Å and c = 18.0 ± 0.20 Å. The powder diffraction data of the EMM-64 material was also analyzed for structure solution by powder XRD, applying the FOCUS algorithm, and was shown to have a 3D 8-ring channel system and a chiral framework.

### Examples 2.12-2.15 - Synthesis of EMM-64 at 150°C, with lower H₂O/Si molar ratios

These Examples were carried out under similar conditions as Example 2.02, at 150°C, except at H₂O/Si molar ratios from 1 to 4. Each Experiment produced pure EMM-64 after 5-7 days of heating at 150°C, as identified by their XRD patterns.

Figure 4 shows SEM images of the as-synthesized products of Example 2.12. The 4-fold symmetry of the crystals are apparent. A sample of the product of Example 2.12 was submitted for adsorption tests. The micropore volume (V_{micro}) was 0.13 cc/g and the n-hexane uptake was very slow, reaching only 4.6 mg/g.

### Examples 2.16-2.21 - Synthesis of EMM-64 in the presence of aluminum sources, with varying Si/Al molar ratios

These Examples were carried out under similar conditions as Example 2.02, at 150°C with H₂O/Si molar ratio of 4, except in the presence of aluminum sources, with varying Si/Al molar ratios of 100, 30 and 20. These Examples used two different aluminum sources: aluminum nitrate (15 wt% solution) in Examples 2.16-2.18 and MS-25 amorphous silica-alumina (65.5% silica / 22% alumina) in Examples 2.19-2.21. Each Example produced pure EMM-64 after 6-7 days of heating at 150°C, as identified by their XRD patterns.

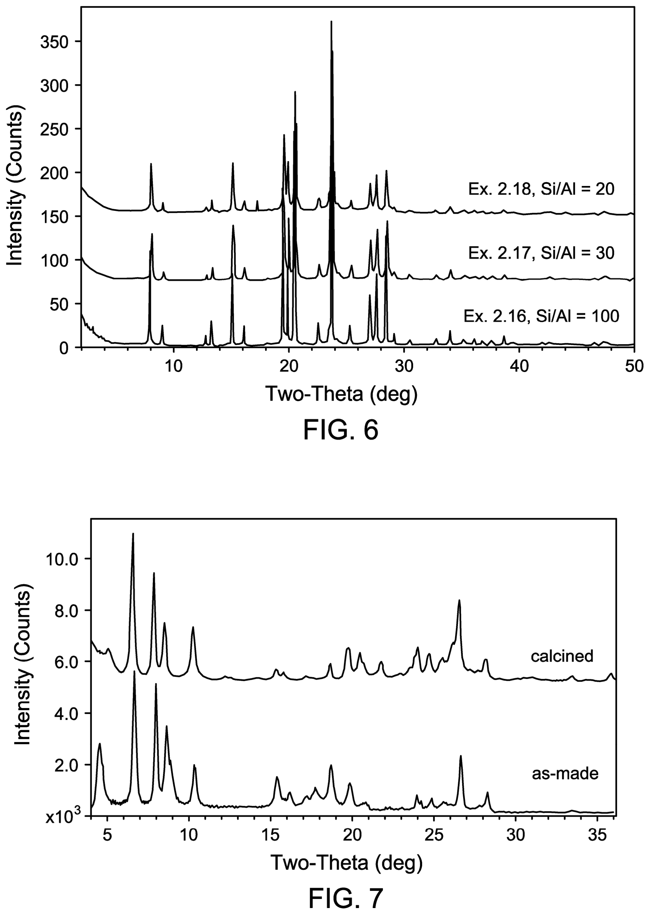

Figure 5 shows SEM images of the as-synthesized products of Examples 2.16, 2.17 and 2.18. As can be seen, the effect of increasing amounts of aluminum is two-fold: the crystallites become smaller with increasing Al content, and they also become less well-faceted and more intergrown. Figure 6 (Examples 2.16, 2.17 and 2.18) shows how increasing aluminum content leads to broader diffraction peaks in the powder XRDs as the individual crystallites become smaller.

**Table 8**

| **Ex.** | **Si/Al** | **H₂O/Si** | **Q/Si** | **OH/Si** | **HF/Si** | **T (°C)** | **t (d)** | **Product** |
|---|---|---|---|---|---|---|---|---|
| 2.01 | - | 4 | 0.50 | 0.50 | 0.50 | 150 | 10 | EMM-64 |
| 2.02 | - | 4 | 0.50 | 0.50 | 0.50 | 150 | 14 | EMM-64 |
| 2.03 | - | 20 | 0.50 | 0.50 | 0.50 | 150 | 14 | EMM-64 |
| 2.04 | - | 30 | 0.50 | 0.50 | 0.50 | 150 | 14 | EMM-64 |
| 2.05 | - | 50 | 0.50 | 0.50 | 0.50 | 150 | 14 | EMM-64 |
| 2.06 | - | 10 | 0.50 | 0.50 | 0.50 | 130 | 13 | EMM-64 |
| 2.07 | - | 4 | 0.50 | 0.50 | 0.50 | 130 | 27 | mostly amorphous, minor EMM-64 |
| 2.08 | - | 7 | 0.50 | 0.50 | 0.50 | 130 | 27 | EMM-64 |
| 2.09 | - | 20 | 0.50 | 0.50 | 0.50 | 130 | 27 | EMM-64 |
| 2.10 | - | 30 | 0.50 | 0.50 | 0.50 | 130 | 27 | EMM-64 + amorphous |
| 2.11 | - | 50 | 0.50 | 0.50 | 0.50 | 130 | 27 | EMM-64 + amorphous |
| 2.12 | - | 1 | 0.50 | 0.50 | 0.50 | 150 | 5 | EMM-64 |
| 2.13 | - | 2 | 0.50 | 0.50 | 0.50 | 150 | 5 | EMM-64 |
| 2.14 | - | 4 | 0.50 | 0.50 | 0.50 | 150 | 5 | EMM-64 |
| 2.15 | - | 3 | 0.50 | 0.50 | 0.50 | 150 | 7 | EMM-64 |
| 2.16 | 100 | 4 | 0.50 | 0.50 | 0.50 | 150 | 6 | EMM-64 |
| 2.17 | 30 | 4 | 0.50 | 0.50 | 0.50 | 150 | 6 | EMM-64 |
| 2.18 | 20 | 4 | 0.50 | 0.50 | 0.50 | 150 | 6 | EMM-64 |
| 2.19 | 100 | 4 | 0.50 | 0.50 | 0.50 | 150 | 6 | EMM-64 |
| 2.20 | 30 | 4 | 0.50 | 0.50 | 0.50 | 150 | 6 | EMM-64 |
| 2.21 | 20 | 4 | 0.50 | 0.50 | 0.50 | 150 | 6 | EMM-64 |

**Table 9**

| **Degree 2-theta** | **d-spacing (Å)** | **Relative intensity [100 x I/(Io)]** |
|---|---|---|
| 8.11 | *10.90* | 100 |
| 9.10 | 9.71 | 11.5 |
| 12.86 | 6.88 | 13.8 |
| 13.41 | 6.60 | 23.7 |
| 15.20 | 5.82 | 40.7 |
| 16.21 | 5.46 | 4.2 |
| 19.74 | 4.49 | 27.6 |
| 19.94 | 4.45 | 18.3 |
| 20.65 | 4.30 | 49.6 |
| 22.66 | 3.92 | 5.8 |
| 23.78 | 3.74 | 57.2 |
| 24.38 | 3.65 | 2.5 |
| 25.57 | 3.48 | 3.7 |
| 27.11 | 3.29 | 13.1 |
| 27.70 | 3.22 | 21.1 |
| 28.70 | 3.11 | 23.3 |
| 29.21 | 3.06 | 5.3 |
| 30.59 | 2.92 | 2.5 |
| 32.54 | 2.75 | 0.7 |
| 32.82 | 2.73 | 2.9 |
| 34.19 | 2.62 | 6.4 |
| 35.28 | 2.54 | 4.4 |
| 35.80 | 2.51 | 1.4 |
| 36.32 | 2.47 | 2.8 |
| 36.85 | 2.44 | 5.2 |
| 37.87 | 2.37 | 2.4 |
| 38.82 | 2.32 | 5.6 |
| 39.37 | 2.29 | 1 |
| 39.80 | 2.26 | 1.3 |
| 40.07 | 2.25 | 1.9 |
| 41.22 | 2.19 | 2.2 |

**Table 10**

| **Degree 2-theta** | **d-spacing (Å)** | **Relative intensity [100 x I/(Io)]** |
|---|---|---|
| 8.08 | 10.93 | 29.9 |
| 9.11 | 9.70 | 5.2 |
| 12.87 | 6.87 | 2.1 |
| 13.34 | 6.63 | 5.4 |
| 15.20 | 5.83 | 19.8 |
| 16.17 | 5.48 | 5.4 |
| 18.24 | 4.86 | 0.7 |
| 19.54 | 4.54 | 35.5 |
| 19.94 | 4.45 | 24.2 |
| 20.56 | 4.32 | 63.1 |
| 22.62 | 3.93 | 7.6 |
| 23.45 | 3.79 | 3 |
| 23.79 | 3.74 | 100 |
| 24.29 | 3.66 | 2.8 |
| 25.34 | 3.51 | 6.2 |
| 27.12 | 3.29 | 17.9 |
| 27.49 | 3.24 | 0.7 |
| 27.69 | 3.22 | 26.5 |
| 28.50 | 3.13 | 30.1 |
| 28.99 | 3.08 | 0.9 |
| 29.19 | 3.06 | 4.4 |
| 30.54 | 2.92 | 2.7 |
| 32.29 | 2.77 | 0.6 |
| 32.86 | 2.72 | 4.1 |
| 34.03 | 2.63 | 8.1 |
| 34.90 | 2.57 | 1.3 |
| 35.18 | 2.55 | 2.6 |
| 35.42 | 2.53 | 3.9 |
| 35.77 | 2.51 | 1.5 |
| 36.09 | 2.49 | 4.2 |
| 36.50 | 2.46 | 1.1 |
| 36.87 | 2.44 | 3.4 |
| 37.57 | 2.39 | 4 |
| 38.30 | 2.35 | 1.8 |
| 38.76 | 2.32 | 8 |
| 39.37 | 2.29 | 3.7 |

### Example 3 - Synthesis of EMM-65

The molar ratios and conditions used for the syntheses of Examples 3.01-3.05, as well as the resulting products, are detailed below and summarized in Table 11.

### Example 3.01 - Synthesis of EMM-65

EMM-65 was first observed from a synthesis performed in a 1.5 mL stainless steel reactor tumbled at 160°C for 7 days, from a synthesis mixture having the following composition in terms of molar ratios: H₂O/Si = 36, Q/Si = 0.20, Na/Si = 0.10, OH/Si = 0.30, and Si/B = 20, using 1-methyl-6,7-dihydro-5H-cyclopenta[b]pyridine-1-ium hydroxide as prepared in Example 1 as structure directing agent (Q) (7.4 wt% solution). The silica source was Aerodisp W 7330N (30 wt% colloidal silica suspension). The boron source was boric acid (3.047 wt% solution). The M source was NaOH (10 wt% solution).

The product was isolated by centrifugation, resuspension in deionized water, and then another centrifugation. This process was repeated three times and then the product was dried at 90°C. The as-synthesized material was then calcined to 600°C within a box furnace via the following procedure. The sample was exposed to flowing nitrogen for two hours at room temperature, followed by a ramp from room temperature to 400°C over a two-hour period while remaining under nitrogen flow. The temperature then remained at 400°C for 15 minutes and then the atmosphere was switched from flowing nitrogen to flowing dried air. The temperature was then ramped from 400°C to 600°C over a 1-hour period. The temperature remained at 600°C for 2 hours and then the box furnace was allowed to cool.

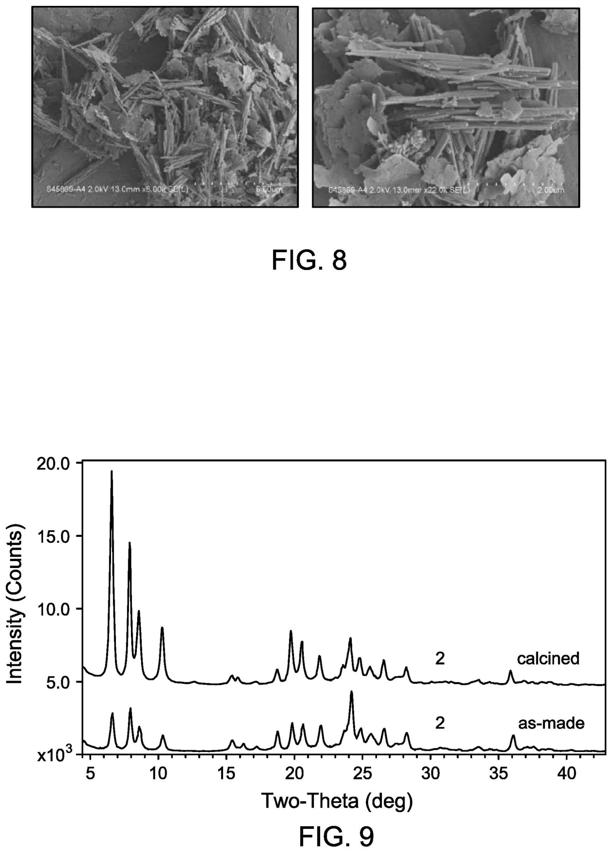

XRD analysis of the as-synthesized and as-calcined materials showed the material to have unique powder XRD patterns which could not be matched with any known zeolite and were designated as pure as-synthesized and as-calcined EMM-65 products. A layered phase impurity is manifested by a peak around 4.5 °20. Upon calcination, this feature remains but loses intensity and moves to 5.1 °20. The position of this peak is consistent with the presence of Kenyaite. Figure 8 shows SEM images of the as-synthesized product. The needles or laths are due to the EMM-65, and the plates are due to the layered phase impurity.

### Example 3.02 - Larger scale synthesis of EMM-65 (45 mL)

This Example was a larger-scale reproduction of Example 3.01, carried out in a 45 mL stainless steel Parr autoclave with a PTFE liner. 6.7 g Aerodisp W 7330N, 13.7 g 1-methyl-6,7-dihydro-5H-cyclopenta[b]pyridine-1-ium hydroxide (7.4 wt% solution), 3 g boric acid solution (3.047 wt%), 1.34 g NaOH solution (10 wt%), and 0.26 g deionized water were added together and mixed to create a uniform suspension. The mixture was then sealed within the autoclave reactor, placed within a spit in a convection oven, and heated at 160°C for 7 days under tumbling conditions (about 40 rpm). After washing with deionized water (about 250 mL) under filtration, the solid product was dried in an oven at 90°C overnight to yield 2.2 g of dried product.

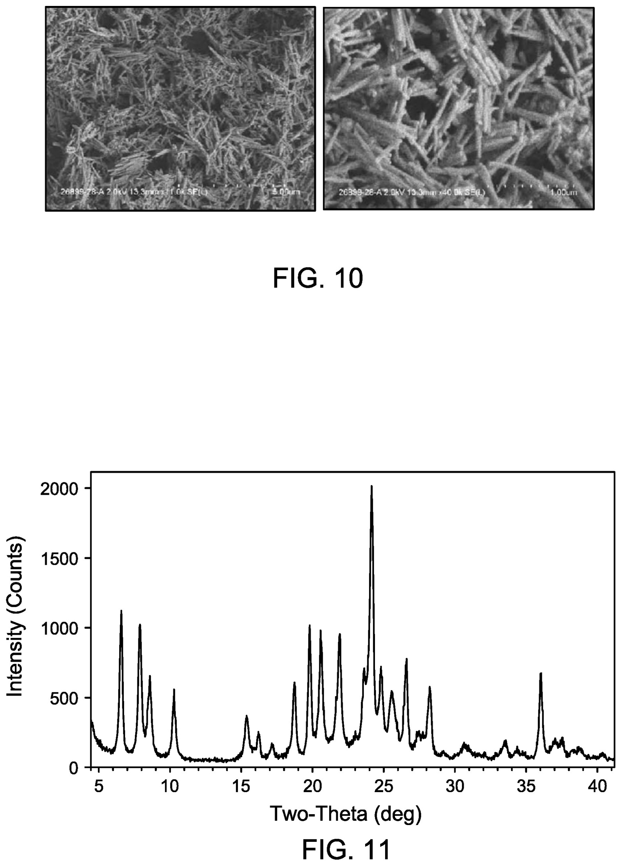

Powder XRD patterns of the as-synthesized and as-calcined materials, as illustrated in Figure 9, showed the product to be pure EMM-65. Tables 12 and 13 below show the peak positions and intensities for powder XRD patterns of the as-calcined and as-made EMM-65 products of Example 3.02. Figure 10 shows SEM images of the as-synthesized EMM-65 product, composed of needles or laths. The SEM images also show the absence of the plates observed in small scale Example 3.01.

### Example 3.03 - Larger scale synthesis of EMM-65 (125 mL)

This Experiment was a larger-scale reproduction of Example 3.02, carried out in a 125 mL steel Parr autoclave with PTFE liner. This synthesis used 18.8 g of Aerodisp W 7330N. Pure EMM-65 was obtained after 7 days of heating at 160°C, as identified by its XRD pattern. Elemental analysis by inductively coupled plasma (ICP) method of the as-calcined product showed the material to have a Si/B atomic ratio of 22.4 and a Na/Si atomic ratio of 0.005.

### Example 3.04 - Larger scale synthesis of EMM-65 (300 mL)

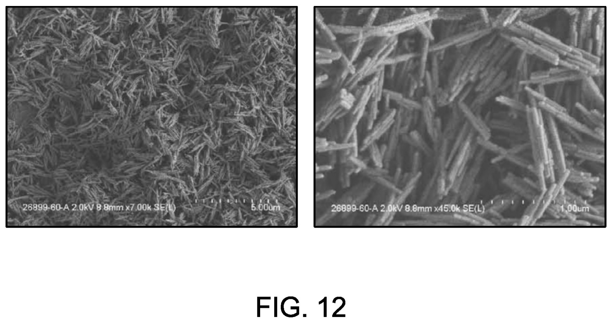

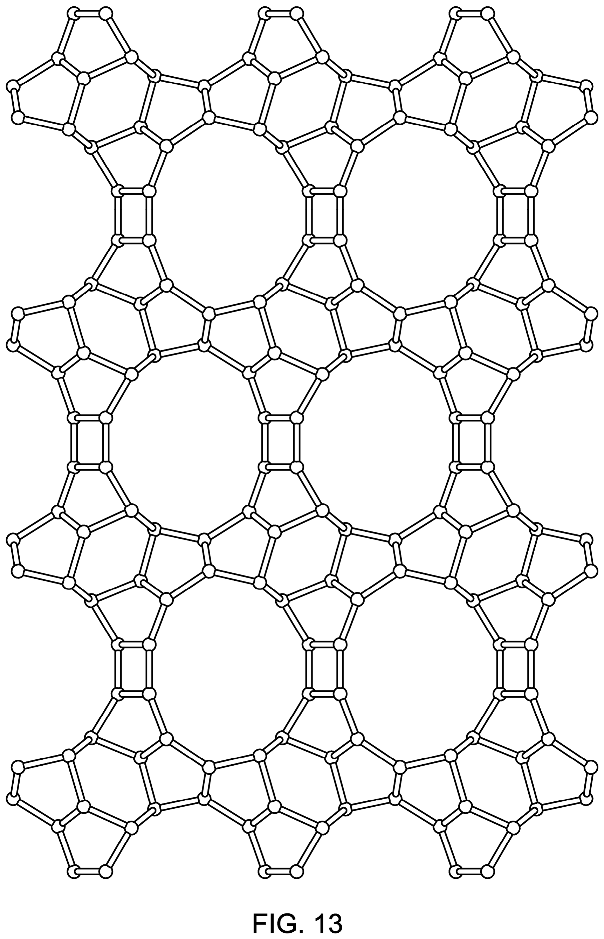

This Experiment was a larger-scale reproduction of Example 3.02, carried out in a 300 mL overhead stirred steel Parr autoclave. This synthesis used 64.4 g of Aerodisp W 7330N and 0.19 g of the product of Example 3.03 as seeds. Pure EMM-65 was obtained after 8 days of heating at 160°C, as identified by the XRD pattern of the as-synthesized product, illustrated in Figure 11. The sharper features of the powder XRD pattern compared to those of Figures 7 and 9 are due to thicker crystal dimensions . Figure 12 shows SEM images of the as-synthesized EMM-65 product. The powder diffraction data of the as-synthesized and as-calcined EMM-65 products could be indexed in orthorhombic space group *Pmmn2* with unit cell dimensions a = 5.15 ± 0.20 Å, b = 27.3 ± 0.20 Å, and c = 11.4 ± 0.20 Å. A sample of the EMM-65 product was analyzed for structure solution by electron diffraction and was shown to have a 1D 12-ring channel system with pore dimensions of 7.9 ± 0.20 Å by 6.0 ± 0.20 Å. Figure 13 shows a projection of EMM-65 along its 12-ring pores.

The as-synthesized material was calcined at 500°C for 16 hours, ammonium ion-exchanged in a batch system with a 1M ammonium nitrate solution, and then calcined again at 500°C for 16 hours. The BET surface area (S_{BET}) of the material was 387 m²/g and its micropore volume (V_{micro}) was 0.13 cc/g.

### Example 3.05 - Synthesis of EMM-65 using KOH as source of M

This Experiment was carried out under similar conditions as Example 3.02, except KOH was used instead of NaOH as source of M, and 0.0145 g of the product of Example 3.03 were added as seeds. This synthesis used 4.8 g Aerodisp W 7330N. After 12 days of heating at 160°C, pure EMM-65 was obtained, as identified by its XRD pattern. The powder XRD had broader features than previous samples because the crystallites were thinner.

**Table 11**

| **Ex.** | **Si/B** | **H₂O/Si** | **Q/Si** | **OH/Si** | **M/Si** | **T (°C)** | **t (d)** | **Product** |
|---|---|---|---|---|---|---|---|---|
| 3.01 | 20 | 36 | 0.20 | 0.30 | 0.10 (Na) | 160 | 7 | EMM-65 |
| 3.02 | 20 | 36 | 0.20 | 0.30 | 0.10 (Na) | 160 | 7 | EMM-65 |
| 3.03 | 20 | 36 | 0.20 | 0.30 | 0.10 (Na) | 160 | 7 | EMM-65 |
| 3.04 | 20 | 36 | 0.20 | 0.30 | 0.10 (Na) | 160 | 8 | EMM-65 |
| 3.05 | 20 | 36 | 0.20 | 0.30 | 0.10 (K) | 160 | 12 | EMM-65 |

**Table 12**

| **Degree 2-theta** | **d-spacing (Å)** | **Relative intensity [100 x I/(Io)]** |
|---|---|---|
| 6.583 | 13.42 | 100 |
| 7.908 | 11.17 | 62.8 |
| 8.562 | 10.32 | 40.2 |
| 10.301 | 8.58 | 31.5 |
| 12.658 | 6.99 | 1.2 |
| 15.392 | 5.75 | 5.3 |
| 15.859 | 5.58 | 3.4 |
| 17.145 | 5.17 | 1 |
| 18.725 | 4.74 | 6.7 |
| 19.756 | 4.49 | 28.7 |
| 20.521 | 4.32 | 24.2 |
| 21.834 | 4.07 | 15.9 |
| 22.987 | 3.87 | 1.7 |
| 23.567 | 3.77 | 9.3 |
| 24.068 | 3.69 | 27.5 |
| 24.764 | 3.59 | 15 |
| 25.549 | 3.48 | 11.5 |
| 26.538 | 3.36 | 11.3 |
| 27.359 | 3.26 | 1.3 |
| 27.761 | 3.21 | 4 |
| 28.212 | 3.16 | 7.5 |
| 29.2 | 3.06 | 0.2 |
| 30.053 | 2.97 | 0.7 |
| 30.596 | 2.92 | 1.9 |
| 31.069 | 2.88 | 1.2 |
| 31.517 | 2.84 | 1.2 |
| 31.989 | 2.80 | 0.5 |
| 33.373 | 2.68 | 3.8 |
| 34.341 | 2.61 | 1.2 |
| 35.854 | 2.50 | 6.2 |
| 36.597 | 2.45 | 1.1 |

**Table 13**

| **Degree 2-theta** | **d-spacing (Å)** | **Relative intensity [100 x I/(Io)]** |
|---|---|---|
| 6.64 | 13.30 | 49.4 |
| 7.97 | 11.09 | 55.7 |
| 8.63 | 10.24 | 37.5 |
| 10.35 | 8.54 | 22.7 |
| 15.44 | 5.73 | 22.2 |
| 16.24 | 5.45 | 11.8 |
| 17.21 | 5.15 | 4.7 |
| 18.76 | 4.73 | 26.4 |
| 19.85 | 4.47 | 31.3 |
| 20.61 | 4.31 | 44.1 |
| 21.93 | 4.05 | 35.6 |
| 23.04 | 3.86 | 1.9 |
| 23.72 | 3.75 | 26.9 |
| 24.18 | 3.68 | 100 |
| 24.84 | 3.58 | 25 |
| 25.64 | 3.47 | 37.5 |
| 26.56 | 3.35 | 32.3 |
| 27.60 | 3.23 | 14 |
| 28.23 | 3.16 | 25.6 |
| 29.14 | 3.06 | 0.9 |
| 30.84 | 2.90 | 13.4 |
| 32.04 | 2.79 | 2.1 |
| 33.47 | 2.68 | 11.2 |
| 34.46 | 2.60 | 4.2 |
| 36.04 | 2.49 | 25.6 |
| 40.30 | 2.24 | 1.8 |
| 37.25 | 2.41 | 16.6 |
| 38.22 | 2.35 | 1.5 |
| 38.69 | 2.33 | 8.7 |

### Example 3.06 - Preparation of aluminum exchanged EMM-65

A portion of the ammonium-exchanged and calcined product of Example 3.04 was heated at 80°C in a 1 M aluminum nitrate solution overnight to exchange the framework B with Al. The product was then dried at 100°C and calcined at 500°C for 16 hours.

The Si/Al atomic ratio of the calcined sample was about 37, as determined by EDX. The alpha value of the calcined sample was 5, its BET surface area (S_{BET}) was 350 m²/g, and its micropore volume (V_{micro}) was 0.14 cc/g. n-hexane, 2,3-dimethylbutane (2,3-DMB), and mesitylene uptakes were determined on calcined materials. The material was placed under a nitrogen stream then the hydrocarbon was introduced through a sparger to saturate the nitrogen stream and the hydrocarbon uptake was determined. Each hydrocarbon was adsorbed at a different temperature: n-hexane was adsorbed at 90°C, 2,3-DMB was adsorbed at 120°C, and mesitylene was adsorbed at 100°C. n-hexane uptake was 50.5 mg/g, 2,3-DMB uptake was 45 mg/g, and mesitylene uptake was 58 mg/g.

### Example 4 - Synthesis of RTH zeolites

The raw materials, molar ratios and conditions used for the syntheses of Examples 4.01-4.11, as well as the resulting products, are detailed below and summarized in Table 14.

### Example 4.01 - Synthesis of RTH zeolite

To a 1.5 mL stainless steel reactor, the following reagents were added in this order while stirring at about 200 rpm: 7.76 µl distilled water, 324.5 µl 1-methyl-6,7-dihydro-5H-cyclopenta[b]pyridin-1-ium hydroxide (7.4 wt% solution) as structure directing agent (Q), 74.54 µl of Ludox LS-30 (30 wt% colloidal silica suspension), 58.13 µl NaOH (10 wt% solution) and 8.40 mg Ultrastable Y (USY, Si/Al = 3). The resulting synthesis mixture had the following composition in terms of molar ratios: H₂O/Si = 45, Si/Al = 20, Q/Si = 0.30, OH/Si = 0.60, M/Si = 0.30. The synthesis mixture was stirred for about 2 hours, then placed in a tumbling oven at about 45 rpm and heated to 160°C for 7 days. The reaction mixture was cooled and the product was recovered by centrifugation, washing with distilled water, and another centrifugation. This process was repeated three times. The product was then dried at 90°C in a vented drying oven. The as-synthesized material was then calcined to 600°C within a box furnace via the following procedure. The sample was exposed to flowing nitrogen for two hours at room temperature, followed by a ramp from room temperature to 400°C over a two-hour period while remaining under nitrogen flow. The temperature then remained at 400°C for 15 minutes and then the atmosphere was switched from flowing nitrogen to flowing dried air. The temperature was then ramped from 400°C to 600°C over a 1-hour period. The temperature remained at 600°C for 2 hours and then the box furnace was allowed to cool.

XRD analysis of the as-calcined material showed the material to have a powder XRD pattern characteristic of aluminosilicate zeolites of RTH framework type. The material further contained a minor amount of quartz.

### Example 4.02 - Reproduction of Example 4.01 with longer heating time of 28 days

This Example was carried out under similar conditions as Example 4.01, except that the synthesis mixture was heated for 28 days rather than 7 days. The resulting product was identified as a mixture of RTH zeolite and quartz (about 50:50).

This result can be explained by the presence of excess silica in the synthesis mixture: Si/Al molar ratio of 20 vs. Si/Al molar ratio of 7 for an ideal composition of the RTH zeolite product with two molecules of structure directing agent (SDA) per cage and therefore two positive charges per cage. This excess silica may be either amorphous, dissolved in the mother liquor, or crystallized as a silica-rich phase, e.g. quartz. Without wishing to be bound by theory, it is believed that, at early times, much of the excess silica remains dissolved in the mother liquor, in particular in view of the rather high OH content of the present synthesis mixture (OH/Si = 0.6). However, with time, the dissolved silica can form quartz. This can also be favored by a drop in the OH content as, with time, SDA may degrade and consume part of said OH content.

### Example 4.03 - Synthesis of RTH zeolite with lower Si/Al molar ratio

This Example was carried out under similar conditions as Example 4.02, except that the synthesis mixture had a Si/Al molar ratio of 10. The resulting product was identified as RTH zeolite, based on its XRD pattern. This confirms the explanation given for the results obtained in Example 4.02.

### Examples 4.04-4.06 - Synthesis of RTH zeolite using Georgia metakaolin as Al source

Example 4.04 was carried out under similar conditions as Example 4.03 but using Georgia metakaolin as Al source. Example 4.05 was carried out under similar conditions as Example 4.04 but at a lower Si/Al molar ratio of 5 and for a shorter heating time of 14 days. Example 4.06 was conducted at similar conditions as Example 4.05 but using KOH rather than NaOH. The resulting products were identified as RTH zeolite, possibly in combination with zeolite of ANA framework-type, based on their XRD patterns.

### Examples 4.07-4.08 - Synthesis of RTH zeolite using sodium aluminate as Al source

Example 4.07 was carried out under similar conditions as Example 4.03 but using sodium aluminate (NaAlO₂, 8.86 wt% solution) as Al source. Example 4.08 was carried out under similar conditions as Example 4.07 but at a lower temperature of 120°C and with a lower NaOH/Si molar ratio of 0.15. The resulting products were identified as RTH zeolite based on their XRD patterns.

### Examples 4.09-4.11 - Alternative examples of RTH zeolite synthesis

Example 4.09 was carried out following the experimental procedure of Example 4.01 but using Aerodisp W 7330N (30 wt% colloidal silica suspension) as Si source and sodium aluminate (NaAlO₂, 8.86 wt% solution) as Al source, and with a lower Si/Al molar ratio of 5. The resulting product was identified as RTH zeolite based on its XRD pattern.

Example 4.10 was carried out in a similar way as Example 4.09 but with varying H₂O/Si, Si/Al and NaOH/Si molar ratios as defined in Table 14, and in the presence of HCl. The resulting product was identified as a mixture of RTH and ANA zeolites based on its XRD pattern.

Example 4.11 was carried out in a similar way as Example 4.09 or 4.10 but at a lower temperature of 120°C, for a longer heating time of 28 days, and with varying molar ratios as defined in Table 14, in the presence of HCl. The resulting product was identified as RTH zeolite based on its XRD pattern.

**Table 14**

| **Ex.** | **Si Source*** | **Al Source*** | **Si/Al** | **H₂O/Si** | **Q/Si** | **OH/Si** | **M/Si** | **Cl/S i** | **T (°C)** | **t (d)** | **Product** |
|---|---|---|---|---|---|---|---|---|---|---|---|
| 4.01 | L | USY | 20 | 45 | 0.30 | 0.60 | 0.30 (Na) | 0 | 160 | 7 | RTH |
| 4.02 | L | USY | 20 | 45 | 0.30 | 0.60 | 0.30 (Na) | 0 | 160 | 28 | 50/50: RTH/ANA |
| 4.03 | L | USY | 10 | 44 | 0.30 | 0.60 | 0.30 (Na) | 0 | 160 | 28 | RTH |
| 4.04 | L | MK | 10 | 45 | 0.30 | 0.60 | 0.30 (Na) | 0 | 160 | 28 | RTH, minor ANA |
| 4.05 | L | MK | 5 | 44 | 0.30 | 0.60 | 0.30 (Na) | 0 | 160 | 14 | 50/50: RTH/ANA |
| 4.06 | L | MK | 5 | 42 | 0.30 | 0.60 | 0.30 (K) | 0 | 160 | 14 | RTH |
| 4.07 | L | SA | 10 | 48 | 0.30 | 0.60 | 0.30 (Na) | 0 | 160 | 28 | RTH |
| 4.08 | L | SA | 10 | 50 | 0.30 | 0.45 | 0.15 (Na) | 0 | 120 | 28 | RTH |
| 4.09 | A | SA | 5 | 43 | 0.30 | 0.60 | 0.30 (Na) | 0 | 160 | 7 | RTH |
| 4.10 | A | SA | 20 | 64 | 0.30 | 1.30 | 1.00 (Na) | 0.30 | 160 | 7 | 50/50: RTH/ANA |
| 4.11 | A | SA | 40 | 56 | 0.20 | 0.90 | 0.70 (Na) | 0.25 | 120 | 28 | RTH |

| | | | | | | | | | | | |
|---|---|---|---|---|---|---|---|---|---|---|---|
| * L = Ludox LS-30, A = Aerodisp W 7330N, MK = metakaolin, Y = Ultrastable Y, SA = sodium aluminate. | | | | | | | | | | | |

Without wishing to be bound by theory, the inventors believe that the RTH zeolite products occlude two molecules of structure directing agent (SDA) per RTH cavity. The molecules may pi-stack. The higher density of extra-framework charge leads to a necessarily higher framework negative charge, and hence RTH is able to crystallize as a pure material from syntheses mixtures with especially low Si/Al molar ratio, e.g. Si/Al molar ratio lower than 15, such as 10 or even 5. In the absence of occluded alkali cations, the ideal Si/Al ratio for two SDAs per cavity is 7. For one single charged cation per cavity, the ideal Si/Al ratio would be 15.

### Comparative Example 5 - Use of 1-methyl-5,6,7,8-tetrahydroquinolinium hydroxide as structure directing agent

Example 3.02 was repeated except that 1-methyl-5,6,7,8-tetrahydroquinolinium hydroxide was used as the structure directing agent (Q) rather than 1-methyl-6,7-dihydro-5H-cyclopenta[b]pyridine-1-ium hydroxide. After 7 days of heating at 160°C, a mixture of SSZ-59 (SFN framework type) and quartz was obtained.

While the present invention has been described and illustrated with reference to particular embodiments, it will be appreciated by those of ordinary skill in the art that the invention lends itself to many different alterations, modifications, and variations not specifically illustrated herein. It will also be apparent to those skilled in the art that when numerical lower limits and numerical upper limits are listed herein, ranges from any lower limit to any upper limit are contemplated. Also, all numerical values within the detailed description herein are modified by "about" the indicated value, and take into account experimental error and variations that would be expected by a person having ordinary skill in the art.

Where in the foregoing description, integers or elements are mentioned which have known, obvious or foreseeable equivalents, then such equivalents are herein incorporated as if individually set forth. Reference should be made to the claims for determining the true scope of the present invention, which should be construed so as to encompass any such equivalents. It will also be appreciated by the reader that integers or features of the invention that are described as preferable, advantageous, convenient or the like are optional and do not limit the scope of the independent claims. Moreover, it is to be understood that such optional integers or features, whilst of possible benefit in some embodiments of the invention, may not be desirable, and may therefore be absent, in other embodiments.

Additionally or alternately, the invention relates to:
Embodiment 1: Use of 1-methyl-6,7-dihydro-5H-cyclopenta[b]pyridine-1-ium cation of Formula I: 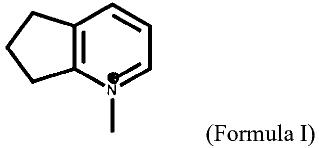 as a structure directing agent for the preparation of zeolites.
Embodiment 2: The use of embodiment 1, wherein the 1-methyl-6,7-dihydro-5H-cyclopenta[b]pyridine-1-ium cation of Formula I is in its hydroxide and/or halide form, preferably in its hydroxide, chloride, bromide, iodide or fluoride form, more preferably in its hydroxide or fluoride form, most preferably in its hydroxide form.
Embodiment 3: The use of embodiment 1 or 2, for the preparation of EMM-64, EMM-65 or aluminosilicate RTH framework type zeolite.
Embodiment 4: A crystalline material designated EMM-64, having, in its as-calcined form, an X-ray diffraction pattern including at least 9, preferably at least 10, more preferably at least 11, most preferably all of the peaks selected from Table 1:

**Table 1**

| **degree 2-theta (±0.20)** | **relative intensity [100 x I/(Io)]** |
|---|---|
| 8.11 | 60-100 |
| 9.10 | 10-20 |
| 12.86 | 10-20 |
| 13.41 | 10-30 |
| 15.20 | 30-60 |
| 19.74 | 20-40 |
| 19.94 | 10-30 |
| 20.65 | 40-70 |
| 23.78 | 40-70 |
| 27.11 | 10-20 |
| 27.70 | 10-30 |
| 28.70 | 10-30 |

Embodiment 5: The material of embodiment 4 having a molecular formula of Formula II:

(m)X₂O₃:YO₂ (Formula II),

wherein 0≤m≤0.025, X is a trivalent element, and Y is a tetravalent element; in particular wherein X includes one or more of Al, B, Fe and Ga, preferably X comprises or is Al and/or B more preferably Al, and Y includes one or more of Si, Ge, Sn, Ti and Zr, preferably Y comprises or is Si.
Embodiment 6: A crystalline material designated EMM-64, having, in its as-synthesized form, an X-ray diffraction pattern including at least 7, preferably at least 8, more preferably all of the peaks selected from Table 3:

**Table 3**

| **degree 2-theta (±0.20)** | **relative intensity [100 x I/(Io)]** |
|---|---|
| 8.08 | 20-40 |
| 15.20 | 10-30 |
| 19.54 | 30-60 |
| 19.94 | 10-30 |
| 20.56 | 50-80 |
| 23.79 | 60-100 |
| 27.12 | 10-30 |
| 27.69 | 20-40 |
| 28.50 | 20-40 |

Embodiment 7: The material of embodiment 6 having a molecular formula of Formula III:

(n)Q : (m)X₂O₃: YO₂ (Formula III),

wherein 0≤n≤0.2, 0≤m≤0.025, Q is 1-methyl-6,7-dihydro-5H-cyclopenta[b]pyridine-1-ium cation of Formula I, X is a trivalent element, and Y is a tetravalent element, 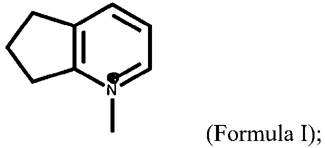 in particular wherein X includes one or more of Al, B, Fe and Ga, preferably X comprises or is Al and/or B more preferably Al, and Y includes one or more of Si, Ge, Sn, Ti and Zr, preferably Y comprises or is Si.
Embodiment 8: The crystalline material of any one of embodiments 4 to 7, having a framework defined by the following connectivities in Table 2 for the tetrahedral (T) atoms in the unit cell, the tetrahedral (T) atoms being connected by bridging atoms:

**Table 2**

| **Topol. equiv. T-atom^{a}** | **Coordination Sequence N₁ to N₁₂** | | | | | | | | | | | |
|---|---|---|---|---|---|---|---|---|---|---|---|---|
| T1 | 4 | 11 | 24 | 39 | 66 | 98 | 129 | 162 | 209 | 263 | 327 | 385 |
| T2 | 4 | 12 | 24 | 42 | 67 | 83 | 128 | 171 | 217 | 262 | 322 | 385 |
| T3 | 4 | 12 | 25 | 41 | 64 | 95 | 132 | 168 | 209 | 265 | 330 | 384 |
| T4 | 4 | 11 | 23 | 42 | 67 | 94 | 126 | 167 | 213 | 265 | 317 | 380 |

| | | | | | | | | | | | | |
|---|---|---|---|---|---|---|---|---|---|---|---|---|
| ^{a} Topologically equivalent atom positions have the same "T-type" symbol. ^{b} Size and number of smallest ring on each angle of the T-atom (M. O'Keeffe and S.T. Hyde, Zeolites, 19, 370 (1997)). | | | | | | | | | | | | |

Embodiment 9: A method for preparing the EMM-64 crystalline material of any one of embodiments 4 to 8, comprising:
(a) preparing a synthesis mixture comprising water, a source of an oxide of tetravalent element (Y), optionally a source of an oxide of trivalent element (X), a structure directing agent (Q), a source of fluoride ions (F), optionally a source of hydroxide ions (OH), and optionally a source of alkali and/or alkaline earth metal element (M),
   wherein the structure directing agent (Q) comprises 1-methyl-6,7-dihydro-5H-cyclopenta[b]pyridine-1-ium cation of Formula I: 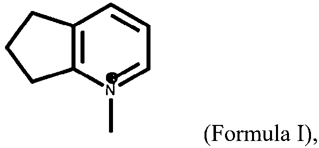 and wherein the synthesis mixture comprises fluoride ions (F) in a F/Y molar ratio of at least 0.1;
(b) heating said synthesis mixture under crystallization conditions including a temperature of from 100 to 200°C for a time sufficient to form crystals of said crystalline material;
(c) recovering at least a portion of the crystalline material from step (b); and
(d) optionally treating the crystalline material recovered in step (c) to remove at least part of the structure directing agent (Q).
Embodiment 10: The method of embodiment 9, wherein the synthesis mixture has the following composition in terms of molar ratios:

| **Molar ratios** | **Typical range** | **Preferred range** | **More preferred range** |
|---|---|---|---|
| Y/X | 10 - infinity | 20 - 500 (if X present) | 20 - 100 (if X present) |
| Q/Y | 0.01 - 1.0 | 0.05 - 1.0 | 0.1 - 0.8 |
| F/Y | 0.1 - 1.0 | 0.2 - 0.8 | 0.3 - 0.7 |
| OH/Y | 0 - 1.5 | 0.05 - 1.0 (if OH present) | 0.1 - 0.8 (if OH present) |
| M/Y | 0 - 1.5 | 0.05 - 1.0 (if M present) | 0.1 - 0.8 (if M present) |
| H₂O/Y | 1 - 100 | 1 - 75 | 1 - 50 |

Embodiment 11: A crystalline material designated EMM-65, having, in its calcined form, an X-ray diffraction pattern including at least 8, preferably at least 9, more preferably at least 10, most preferably all of the peaks selected from Table 4:

**Table 4**

| **degree 2-theta (±0.20)** | **relative intensity [100 x I/(Io)]** |
|---|---|
| 6.58 | 60-100 |
| 7.91 | 50-80 |
| 8.56 | 30-60 |
| 10.30 | 20-40 |
| 19.76 | 20-40 |
| 20.52 | 10-30 |
| 21.83 | 10-30 |
| 24.07 | 20-40 |
| 24.76 | 10-20 |
| 25.55 | 10-20 |
| 26.54 | 10-20 |

Embodiment 12: The material of embodiment 11 having a molecular formula of Formula IV:

(m)B₂O₃:SiO₂ (Formula IV),

wherein 0.005≤m≤0.05.
Embodiment 13: A crystalline material designated EMM-65, having, in its as-synthesized form, an X-ray diffraction pattern including at least 18, preferably at least 19, more preferably at least 20, most preferably all of the peaks selected from Table 6:

**Table 6**

| **degree 2-theta (±0.20)** | **relative intensity [100 x I/(Io)]** |
|---|---|
| 6.64 | 30-60 |
| 7.97 | 40-70 |
| 8.63 | 30-60 |
| 10.35 | 10-30 |
| 15.44 | 10-30 |
| 16.24 | 10-20 |
| 18.76 | 20-40 |
| 19.85 | 20-40 |
| 20.61 | 30-60 |
| 21.93 | 30-60 |
| 23.72 | 20-40 |
| 24.18 | 60-100 |
| 24.84 | 10-30 |
| 25.64 | 30-60 |
| 26.56 | 20-40 |
| 27.60 | 10-20 |
| 28.23 | 20-40 |
| 30.84 | 10-20 |
| 33.47 | 10-20 |
| 36.04 | 20-40 |
| 37.25 | 10-30 |

Embodiment 14: The material of embodiment 13 having a molecular formula of Formula V:

(n)Q:(m)B₂O₃: SiO₂ (Formula V),

wherein 0≤n≤0.2, 0.005<m≤0.05, and Q is 1-methyl-6,7-dihydro-5H-cyclopenta[b]pyridine-1-ium cation of Formula I, 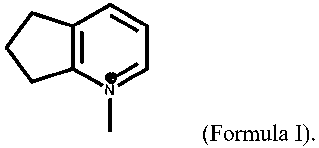 Embodiment 15: The crystalline material of any one of embodiments 11 to 14, having a framework defined by the following connectivities in Table 5 for the tetrahedral (T) atoms in the unit cell, the tetrahedral (T) atoms being connected by bridging atoms:

**Table 5**

| **Topol. equiv. T-atom^{a}** | **Coordination Sequence N₁ to N₁₂** | | | | | | | | | | | |
|---|---|---|---|---|---|---|---|---|---|---|---|---|
| T1 | 4 | 12 | 22 | 37 | 56 | 84 | 121 | 145 | 186 | 321 | 288 | 343 |
| T2 | 4 | 12 | 20 | 37 | 61 | 81 | 114 | 148 | 188 | 238 | 282 | 327 |
| T3 | 4 | 10 | 19 | 34 | 57 | 86 | 111 | 139 | 183 | 236 | 293 | 336 |
| T4 | 4 | 12 | 24 | 37 | 54 | 83 | 117 | 161 | 188 | 225 | 270 | 344 |
| T5 | 4 | 12 | 22 | 36 | 57 | 85 | 117 | 152 | 180 | 229 | 294 | 331 |
| T6 | 4 | 12 | 23 | 41 | 58 | 78 | 112 | 156 | 199 | 231 | 266 | 332 |
| T7 | 4 | 10 | 20 | 36 | 58 | 83 | 113 | 147 | 182 | 230 | 287 | 343 |

| | | | | | | | | | | | | |
|---|---|---|---|---|---|---|---|---|---|---|---|---|
| ^{a} Topologically equivalent atom positions have the same "T-type" symbol. ^{b} Size and number of smallest ring on each angle of the T-atom (M. O'Keeffe and S.T. Hyde, Zeolites, 19, 370 (1997)). | | | | | | | | | | | | |

Embodiment 16: A method for preparing the EMM-65 crystalline material of any one of embodiments 11 to 15, comprising:
(a) preparing a synthesis mixture comprising water, a source of silica, a source of boron (B), a structure directing agent (Q), optionally a source of hydroxide ions (OH), and optionally a source of alkali and/or alkaline earth metal element (M),
   wherein the structure directing agent (Q) comprises 1-methyl-6,7-dihydro-5H-cyclopenta[b]pyridine-1-ium cation of Formula I: 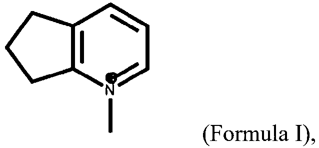 and wherein the synthesis mixture comprises fluoride ions (F) in a F/Si molar ratio of less than 0.1;
(b) heating said synthesis mixture under crystallization conditions including a temperature of from 100°C to 200°C for a time sufficient to form crystals of said crystalline material;
(c) recovering at least a portion of the crystalline material from step (b); and
(d) optionally treating the crystalline material recovered in step (c) to remove at least part of the structure directing agent (Q),
(e) optionally exchanging at least a portion of the boron atoms in the framework silicate with aluminum atoms.
Embodiment 17: The method of embodiment 16, wherein the synthesis mixture has the following composition in terms of molar ratios:

| **Molar ratios** | **Typical range** | **Preferred range** | **More preferred range** |
|---|---|---|---|
| Si/B | 10 - 100 | 15 - 75 | 20 - 50 |
| Q/Si | 0.01 - 1.0 | 0.05 - 1.0 | 0.1 - 0.8 |
| F/Si | 0 - <0.1 | 0 - 0.05 | 0-0.01 |
| OH/Si | 0 - 1.5 | 0.05 - 1.0 (if OH present) | 0.1 - 0.8 (if OH present) |
| M/Si | 0 - 1.5 | 0.05 - 1.0 (if M present) | 0.05 - 0.8 (if M present) |
| H₂O/Si | 1 - 100 | 1 - 75 | 1 - 50 |

Embodiment 18: A method for preparing an aluminosilicate molecular sieve of RTH framework type, comprising:
(a) preparing a synthesis mixture comprising water, a source of silica, a source of alumina, a structure directing agent (Q), optionally a source of hydroxide ions (OH), and a source of alkali and/or alkaline earth metal element (M),
   wherein the structure directing agent (Q) comprises 1-methyl-6,7-dihydro-5H-cyclopenta[b]pyridine-1-ium cation of Formula I: 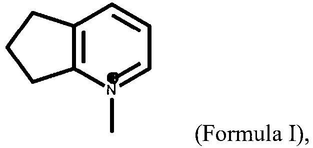 and wherein the synthesis mixture comprises fluoride ions (F) in a F/Si molar ratio of less than 0.1;
(b) heating said synthesis mixture under crystallization conditions including a temperature of from 100°C to 200°C for a time sufficient to form crystals of said crystalline material;
(c) recovering at least a portion of the crystalline material from step (b); and
(d) optionally treating the crystalline material recovered in step (c) to remove at least part of the structure directing agent (Q).
Embodiment 19: The method of embodiment 18, wherein the synthesis mixture has the following composition in terms of molar ratios:

| **Molar ratios** | **Typical range** | **Preferred range** | **More preferred range** |
|---|---|---|---|
| Si/B | 10 - 100 | 15 - 75 | 20 - 50 |
| Q/Si | 0.01 - 1.0 | 0.05 - 1.0 | 0.1 - 0.8 |
| F/Si | 0 - <0.1 | 0 - 0.05 | 0 - 0.01 |
| OH/Si | 0 - 2.0 | 0.1 - 1.5 (if OH present) | 0.2 - 1.3 (if OH present) |
| M/Si | 0.1 - 2.0 | 0.1 - 1.5 | 0.15 - 1.0 |
| H₂O/Si | 1 - 100 | 10 - 80 | 30 - 70 |

Embodiment 20: An aluminosilicate molecular sieve of RTH framework type obtainable by the method of embodiment 18 or 19.
Embodiment 21: An aluminosilicate molecular sieve of RTH framework type having 1-methyl-6,7-dihydro-5H-cyclopenta[b]pyridine-1-ium cation of Formula I: 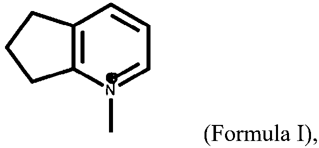 and within its pore structure.
Embodiment 22: An aluminosilicate molecular sieve of RTH framework type having a Si/Al molar ratio of less than 10, preferably from 5 to less than 10.
Embodiment 23: A process of converting an organic compound to a conversion product comprises contacting the organic compound with the crystalline material of any one of embodiments 4 to 6, 11 to 13, or 20 to 22.

## Claims

1. A method for preparing an aluminosilicate molecular sieve of RTH framework type, comprising:
(a) preparing a synthesis mixture comprising water, a source of silica, a source of alumina, a structure directing agent (Q), optionally a source of hydroxide ions (OH), and a source of alkali and/or alkaline earth metal element (M),
wherein the structure directing agent (Q) comprises 1-methyl-6,7-dihydro-5H-cyclopenta[b]pyridine-1-ium cation of Formula I: 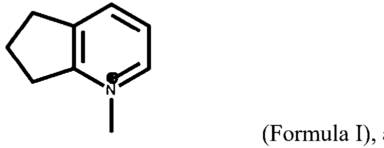 and wherein the synthesis mixture comprises fluoride ions (F) in a F/Si molar ratio of less than 0.1;
(b) heating said synthesis mixture under crystallization conditions including a temperature of from 100°C to 200°C for a time sufficient to form crystals of said crystalline material;
(c) recovering at least a portion of the crystalline material from step (b); and
(d) optionally treating the crystalline material recovered in step (c) to remove at least part of the structure directing agent (Q).

2. The method of claim 1, wherein the synthesis mixture has the following composition in terms of molar ratios:
| **Molar ratios** | **Typical range** | **Preferred range** | **More preferred range** |
|---|---|---|---|
| Si/B | 10 - 100 | 15 - 75 | 20 - 50 |
| Q/Si | 0.01 - 1.0 | 0.05 - 1.0 | 0.1 - 0.8 |
| F/Si | 0 - <0.1 | 0 - 0.05 | 0-0.01 |
| OH/Si | 0 - 2.0 | 0.1 - 1.5 (if OH present) | 0.2 - 1.3 (if OH present) |
| M/Si | 0.1 - 2.0 | 0.1 - 1.5 | 0.15 - 1.0 |
| H₂O/Si | 1 - 100 | 10 - 80 | 30 - 70 |

3. An aluminosilicate molecular sieve of RTH framework type obtainable by the method of claim 1 or 2.

4. An aluminosilicate molecular sieve of RTH framework type having 1-methyl-6,7-dihydro-5H-cyclopenta[b]pyridine-1-ium cation of Formula I: 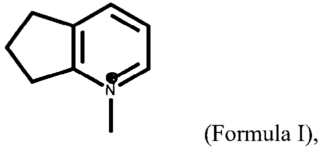 and within its pore structure.

5. An aluminosilicate molecular sieve of RTH framework type having a Si/Al molar ratio of less than 10, preferably from 5 to less than 10.

6. A process of converting an organic compound to a conversion product comprises contacting the organic compound with the molecular sieve of any one of claims 3 to 5.

7. Use of 1-methyl-6,7-dihydro-5H-cyclopenta[b]pyridine-1-ium cation of Formula I: 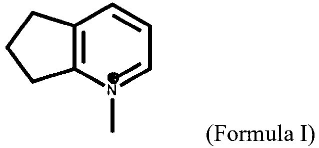 as a structure directing agent for the preparation of an aluminosilicate molecular sieve of RTH framework type.

8. The use of claim 7, wherein the 1-methyl-6,7-dihydro-5H-cyclopenta[b]pyridine-1-ium cation of Formula I is in its hydroxide and/or halide form, preferably in its hydroxide, chloride, bromide, iodide or fluoride form, more preferably in its hydroxide or fluoride form, most preferably in its hydroxide form.
